# EUROPEAN PATENT APPLICATION

(11) **EP 1 491 628 A1**
(43) Date of publication of application: **29.12.2004**
(21) Application number: 03715523.1
(22) Date of filing: 27.03.2003
(51) Int. Cl.: C12N 15/09

(54) **NOVEL GALACTOSE TRANSFERASES, PEPTIDES THEREOF AND NUCLEIC ACID ENCODING THE SAME**

(30) Priority: 29.03.2002 JP 2002094772; 10.07.2002 JP 2002201344
(71) Applicant: National Institute of Advanced Industrial Science and Technology, Tokyo 100-8921 (JP)
(72) Inventor: NARIMATSU, Hisashi, Nat.Inst.Adv.Ind.Sci&Techn., Tsukuba-shi (JP); KUDO, Takashi, Nat.Inst.Advanced Ind.Sci&Tech., Tsukuba-shi (JP); IWASAKI, Hiroko, Nat.Inst.Adv. Ind.Sci&Tech., Tsukuba-shi (JP)
(74) Representative: Keller, Günter, Dr.
(86) International application number: PCT/JP2003/003846
(87) International publication number: WO 2003/083110

(57) **Abstract**

The present inventors succeeded in identifying novel galactosyltransferases, C1Gal-T2 and C1Gal-T3, with a 957-bp ORF and 948-bp ORF, respectively. These enzymes were suggested to be core 1 sugar chain (galactose β1-3 acetylgalactosaminyl α1-R) synthetases. These enzymes and the polynucleotides that encode them are expected to serve as suitable therapeutic agents for diseases caused by abnormal expression or function of these enzymes.

SEQUENCE LISTING

<110> National Institute of Advanced Industrial Science and Technology

<120> NOVEL GALACTOSYLTRANSFERASES, THEIR PEPTIDES, AND
      NUCLEIC ACIDS ENCODING THE SAME

<130> A3-A0201Y1P

<140> 03.715523.1
<141> 2003-03-27

<150> JP 2002-94772
<151> 2002-03-29

<150> JP 2002-201344
<151> 2002-07-10

<160> 24

<170> PatentIn Ver. 2.0

<210> 1
<211> 1471
<212> DNA
<213> Homo sapiens

<220>

<221> CDS

<222> (105)..(1058)

<400> 1
agaacagcct ggtcaggagc gtaacggagt ggtgcgccaa cgtgagagga aacccgtgcg 60
cggctgcgct ttcctgtccc caagccgttc tagacgcggg aaaa atg ctt tct gaa 116
agc agc tcc ttt ttg aag ggt gtg atg ctt gga agc att ttc tgt gct 164
ttg atc act atg cta gga cac att agg att ggt cat gga aat aga atg 212
cac cac cat gag cat cat cac cta caa gct cct aac aaa gaa gat atc 260
ttg aaa att tca gag gat gag cgc atg gag ctc agt aag agc ttt cga 308
gta tac tgt att atc ctt gta aaa ccc aaa gat gtg agt ctt tgg gct 356
gca gta aag gag act tgg acc aaa cac tgt gac aaa gca gag ttc ttc 404
agt tct gaa aat gtt aaa gtg ttt gag tca att aat atg gac aca aat 452
gac atg tgg tta atg atg aga aaa gct tac aaa tac gcc ttt gat aag 500
tat aga gac caa tac aac tgg ttc ttc ctt gca cgc ccc act acg ttt 548
gct atc att gaa aac cta aag tat ttt ttg tta aaa aag gat cca tca 596
cag cct ttc tat cta ggc cac act ata aaa tct gga gac ctt gaa tat 644
gtg ggt atg gaa gga gga att gtc tta agt gta gaa tca atg aaa aga 692
ctt aac agc ctt ctc aat atc cca gaa aag tgt cct gaa cag gga ggg 740
atg att tgg aag ata tct gaa gat aaa cag cta gca gtt tgc ctg aaa 788
tat gct gga gta ttt gca gaa aat gca gaa gat gct gat gga aaa gat 836
gta ttt aat acc aaa tct gtt ggg ctt tct att aaa gag gca atg act 884
tat cac ccc aac cag gta gta gaa ggc tgt tgt tca gat atg gct gtt 932
act ttt aat gga ctg act cca aat cag atg cat gtg atg atg tat ggg 980
gta tac cgc ctt agg gca ttt ggg cat att ttc aat gat gca ttg gtt 1028
ttc tta cct cca aat ggt tct gac aat gac tgagaagtgg tagaaaagcg 1078
tgaatatgat ctttgtatag gacgtgtgtt gtcattattt gtagtagtaa ctacatatcc 1138
aatacagctg tatgtttctt tttcttttct aatttggtgg cactggtata accacacatt 1198
aaagtcagta gtacattttt aaatgagggt ggtttttttc tttaaaacac atgaacattg 1258
taaatgtgtt ggaaagaagt gttttaagaa taataatttt gcaaataaac tattaataaa 1318
tattatatgt gataaattct aaattatgaa cattagaaat ctgtggggca catatttttg 1378
ctgattggtt aaaaaatttt aacaggtctt tagcgttcta agatatgcaa atgatatctc 1438
tagttgtgaa tttgtgatta aagtaaaact ttt 1471

<210> 2

<211> 318

<212> PRT

<213> Homo sapiens

<400> 2

<210> 3

<211> 30

<212> DNA

<213> Artificial Sequence

<220>

<223> Description of Artificial Sequence:Artificially

Synthesized Primer Sequence

<400> 3

gaagatctag aatgcaccac catgagcatc                                    30

<210> 4

<211> 39

<212> DNA

<213> Artificial Sequence

<220>

<223> Description of Artificial Sequence:Artificially

      Synthesized Primer Sequence

<400> 4

ataagaatgc ggccgctcag tcattgtcag aaccatttg                          39

<210> 5

<211> 67

<212> DNA

<213> Artificial Sequence

<220>

<223> Description of Artificial Sequence:Artificially

      Synthesized Primer Sequence

<400> 5

ggggacaagt ttgtacaaaa aagcaggctt agaaggagat agaaccatgc tttctgaaag 60

cagctcc 67

<210> 6

<211> 50

<212> DNA

<213> Artificial Sequence

<220>

<223> Description of Artificial Sequence:Artificially

Synthesized Primer Sequence

<400> 6

ggggaccact ttgtacaaga aagctgggtc tcaatcattg tcagaaccat 50

<210> 7

<211> 19

<212> PRT

<213> Artificial Sequence

<220>

<223> Description of Artificial Sequence:Artificially

Synthesized Peptide Sequence

<400> 7

Val Pro Ser Thr Pro Pro Thr Pro Ser Pro Ser Thr Pro Pro Thr Pro

1               5                   10                  15

Ser Pro Ser

<210> 8

<211> 25

<212> DNA

<213> Artificial Sequence

<220>

<223> Description of Artificial Sequence:Artificially

Synthesized Primer Sequence

<400> 8

gtttgcctga aatatgctgg agtat                                          25

<210> 9

<211> 24

<212> DNA

<213> Artificial Sequence

<220>
<223> Description of Artificial Sequence:Artificially
      Synthesized Primer Sequence

<400> 9
caacagcctt ctactacctg gttg                                          24

<210> 10
<211> 35
<212> DNA
<213> Artificial Sequence

<220>
<223> Description of Artificial Sequence:Artificially
      Synthesized Probe Sequence

<400> 10
cagaaaatgc agaagatgct gatggaaaag atgta                              35

<210> 11
<211> 19
<212> DNA

<213> Artificial Sequence

<220>
<223> Description of Artificial Sequence:Artificially
      Synthesized Primer Sequence

<400> 11
agaaatacac tttcgggaa                                                    19

<210> 12
<211> 20
<212> DNA
<213> Artificial Sequence

<220>
<223> Description of Artificial Sequence:Artificially
      Synthesized Primer Sequence

<400> 12
tgcagtgcta gacatattac                                                   20

<210> 13
<211> 23
<212> DNA

<213> Artificial Sequence

<220>
<223> Description of Artificial Sequence:Artificially
      Synthesized Primer Sequence

<400> 13
gctttcctgt ccccaagccg ttc                                              23

<210> 14
<211> 22
<212> DNA
<213> Artificial Sequence

<220>
<223> Description of Artificial Sequence:Artificially
      Synthesized Primer Sequence

<400> 14
gccccacaga tttctaatgt tc                                               22

<210> 15
<211> 22
<212> DNA

<213> Artificial Sequence

<220>

<223> Description of Artificial Sequence:Artificially

Synthesized Primer Sequence

<400> 15

gtaatcagat tccattggaa gc 22

<210> 16

<211> 69

<212> DNA

<213> Artificial Sequence

<220>

<223> Description of Artificial Sequence:Artificially

Synthesized Primer Sequence

<400> 16

ggggacaagt ttgtacaaaa aagcaggctt cgaaggagat agaaccatgg tttccgctag 60

tgggacatc 69

<210> 17

<211> 56

<212> DNA

<213> Artificial Sequence

<220>

<223> Description of Artificial Sequence:Artificially

Synthesized Primer Sequence

<400> 17

ggggaccact ttgtacaaga aagctgggtc tcagtcattt tctgaaccaa ctggag 56

<210> 18

<211> 948

<212> DNA

<213> Homo sapiens

<220>

<221> CDS

<222> (1)..(948)

<400> 18

atg gtt tcc gct agt ggg aca tca ttt ttt aag ggt atg ttg ctt ggg 48

Met Val Ser Ala Ser Gly Thr Ser Phe Phe Lys Gly Met Leu Leu Gly

1               5                   10                  15

agc att tcc tgg gtt ttg ata act atg ttt ggc caa att cac att cga 96

Ser Ile Ser Trp Val Leu Ile Thr Met Phe Gly Gln Ile His Ile Arg
            20                  25                  30

cac aga ggt caa act caa gac cac gag cac cat cac ctt cgt cca cct 144
His Arg Gly Gln Thr Gln Asp His Glu His His His Leu Arg Pro Pro
        35                  40                  45

aac agg aac gat ttc tta aac act tca aaa gtg ata ctc ttg gag ctc 192
Asn Arg Asn Asp Phe Leu Asn Thr Ser Lys Val Ile Leu Leu Glu Leu
    50                  55                  60

agt aaa agt att cgt gtt ttc tgt atc atc ttt gga gaa tcc gaa gat 240
Ser Lys Ser Ile Arg Val Phe Cys Ile Ile Phe Gly Glu Ser Glu Asp
65                  70                  75                  80

gag agt tac tgg gct gta ctg aaa gag acc tgg acc aaa cac tgt gac 288
Glu Ser Tyr Trp Ala Val Leu Lys Glu Thr Trp Thr Lys His Cys Asp
                85                  90                  95

aaa gca gag ctc tac gat act aaa aat gat aat ttg ttc aat ata gaa 336
Lys Ala Glu Leu Tyr Asp Thr Lys Asn Asp Asn Leu Phe Asn Ile Glu
            100                 105                 110

agt aat gac agg tgg gta cag atg agg acc gct tac aaa tac gtc ttt 384
Ser Asn Asp Arg Trp Val Gln Met Arg Thr Ala Tyr Lys Tyr Val Phe
        115                 120                 125

gaa aag tat ggt gac aac tac aac tgg ttc ttc ctt gca ctt ccc act 432
Glu Lys Tyr Gly Asp Asn Tyr Asn Trp Phe Phe Leu Ala Leu Pro Thr
    130                 135                 140

acg ttt gct gtc att gaa aat tta aag tac ctt ttg ttt aca agg gat 480
Thr Phe Ala Val Ile Glu Asn Leu Lys Tyr Leu Leu Phe Thr Arg Asp
145                 150                 155                 160

gca tcc cag ccc ttc tat ctg ggc cac act gtt ata ttt gga gac ctc 528
Ala Ser Gln Pro Phe Tyr Leu Gly His Thr Val Ile Phe Gly Asp Leu
                165                 170                 175

gaa tac gtg act gtg gaa gga ggg att gtc tta agc aga gag ttg atg 576
Glu Tyr Val Thr Val Glu Gly Gly Ile Val Leu Ser Arg Glu Leu Met
            180                 185                 190

aaa aga ctt aac aga ctt ctc gat aac tct gag acc tgt gca gat caa 624
Lys Arg Leu Asn Arg Leu Leu Asp Asn Ser Glu Thr Cys Ala Asp Gln
        195                 200                 205

agt gtg att tgg aag tta tct gaa gat aag cag ctg gca ata tgc ctg 672
Ser Val Ile Trp Lys Leu Ser Glu Asp Lys Gln Leu Ala Ile Cys Leu
    210                 215                 220

aaa tat gca gga gtt cat gca gaa aat gca gag gat tat gaa gga aga 720

Lys Tyr Ala Gly Val His Ala Glu Asn Ala Glu Asp Tyr Glu Gly Arg
225                 230                 235                 240

gat gta ttt aat aca aaa cca atc gca cag ctt att gaa gag gca ttg 768
Asp Val Phe Asn Thr Lys Pro Ile Ala Gln Leu Ile Glu Glu Ala Leu
                245                 250                 255

tct aat aac cct cag caa gta gta gaa ggc tgc tgt tca gat atg gct 816
Ser Asn Asn Pro Gln Gln Val Val Glu Gly Cys Cys Ser Asp Met Ala
            260                 265                 270

att act ttc aat gga ctg acc ccc caa aag atg gaa gta atg atg tat 864
Ile Thr Phe Asn Gly Leu Thr Pro Gln Lys Met Glu Val Met Met Tyr
        275                 280                 285

ggc ctg tac cgg ctc agg gca ttt gga cac tat ttc aat gac aca ctc 912
Gly Leu Tyr Arg Leu Arg Ala Phe Gly His Tyr Phe Asn Asp Thr Leu
    290                 295                 300

gtt ttc ttg cct cca gtt ggt tca gaa aat gac tga 948
Val Phe Leu Pro Pro Val Gly Ser Glu Asn Asp
305                 310                 315

<210> 19
<211> 315

<212> PRT

<213> Homo sapiens

<400> 19

Met Val Ser Ala Ser Gly Thr Ser Phe Phe Lys Gly Met Leu Leu Gly

1               5                   10                  15

Ser Ile Ser Trp Val Leu Ile Thr Met Phe Gly Gln Ile His Ile Arg

            20                  25                  30

His Arg Gly Gln Thr Gln Asp His Glu His His His Leu Arg Pro Pro

        35                  40                  45

Asn Arg Asn Asp Phe Leu Asn Thr Ser Lys Val Ile Leu Leu Glu Leu

    50                  55                  60

Ser Lys Ser Ile Arg Val Phe Cys Ile Ile Phe Gly Glu Ser Glu Asp

65                  70                  75                  80

Glu Ser Tyr Trp Ala Val Leu Lys Glu Thr Trp Thr Lys His Cys Asp

                85                  90                  95

Lys Ala Glu Leu Tyr Asp Thr Lys Asn Asp Asn Leu Phe Asn Ile Glu

            100                 105                 110

Ser Asn Asp Arg Trp Val Gln Met Arg Thr Ala Tyr Lys Tyr Val Phe

        115                 120                 125

Glu Lys Tyr Gly Asp Asn Tyr Asn Trp Phe Phe Leu Ala Leu Pro Thr

    130                 135                 140

Thr Phe Ala Val Ile Glu Asn Leu Lys Tyr Leu Leu Phe Thr Arg Asp

145                 150                 155                 160

Ala Ser Gln Pro Phe Tyr Leu Gly His Thr Val Ile Phe Gly Asp Leu

                165                 170                 175

Glu Tyr Val Thr Val Glu Gly Gly Ile Val Leu Ser Arg Glu Leu Met
            180                 185                 190
Lys Arg Leu Asn Arg Leu Leu Asp Asn Ser Glu Thr Cys Ala Asp Gln
        195                 200                 205
Ser Val Ile Trp Lys Leu Ser Glu Asp Lys Gln Leu Ala Ile Cys Leu
    210                 215                 220
Lys Tyr Ala Gly Val His Ala Glu Asn Ala Glu Asp Tyr Glu Gly Arg
225                 230                 235                 240
Asp Val Phe Asn Thr Lys Pro Ile Ala Gln Leu Ile Glu Glu Ala Leu
                245                 250                 255
Ser Asn Asn Pro Gln Gln Val Val Glu Gly Cys Cys Ser Asp Met Ala
            260                 265                 270
Ile Thr Phe Asn Gly Leu Thr Pro Gln Lys Met Glu Val Met Met Tyr
        275                 280                 285
Gly Leu Tyr Arg Leu Arg Ala Phe Gly His Tyr Phe Asn Asp Thr Leu
    290                 295                 300
Val Phe Leu Pro Pro Val Gly Ser Glu Asn Asp
305                 310                 315

<210> 20
<211> 34
<212> DNA
<213> Artificial Sequence

<220>

<223> Description of Artificial Sequence:Artificially
      Synthesized Primer Sequence

<400> 20
gcccaagctt cacagaggtc aaactcaaga ccac                                    34

<210> 21
<211> 31
<212> DNA
<213> Artificial Sequence

<220>
<223> Description of Artificial Sequence:Artificially
      Synthesized Primer Sequence

<400> 21
cggaattctc agtcattttc tgaaccaact g                                       31

<210> 22
<211> 22
<212> DNA
<213> Artificial Sequence

<220>

<223> Description of Artificial Sequence:Artificially
Synthesized Primer Sequence

<400> 22
gcctgaaata tgcaggagtt ca 22

<210> 23
<211> 28
<212> DNA
<213> Artificial Sequence

<220>
<223> Description of Artificial Sequence:Artificially
Synthesized Primer Sequence

<400> 23
ggttattaga caatgcctct tcaataag 28

<210> 24
<211> 36
<212> DNA
<213> Artificial Sequence

<220>

<223> Description of Artificial Sequence:Artificially

Synthesized Probe Sequence

<220>

<221> misc_binding

<222> (1)

<223> Label FAM

<220>

<221> misc_binding

<222> (36)

<223> Label MGB-TAMRA

<400> 24

gcagaaaatg cagaggatta tgaaggaaga gatgta 36

## Description

### Technical Field

The present invention relates to novel galactosyltransferases, polynucleotides encoding the same, and uses thereof.

### Background Art

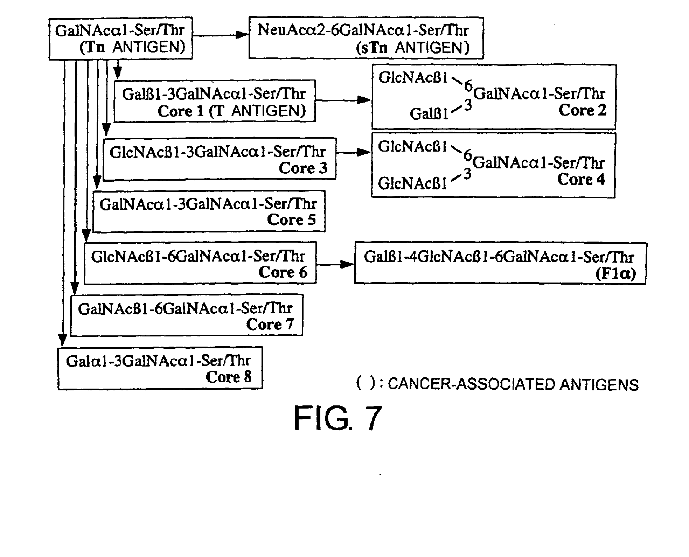

Complex carbohydrates comprise glycoproteins and glycolipids. Depending on the type of peptides and the bonding pattern of their sugars, glycoproteins are broadly classified into three categories: N-linked sugar chains (asparagine-linked sugar chain), O-linked sugar chains (mucin-type sugar chain), and proteoglycans. O-linked sugar chains are those in which the sugars are transferred to serines and threonines through O-glycosidic linkages. Although the primary type of sugar is N-acetylgalactosamine (GalNAc), sugars including fucose (Fuc), mannose (Man), and N-acetylglucosamine (GlcNAc) are also transferred. These sugars are first transferred to peptides, and then the O-linked sugar chains are elongated by various glycosyltransferases. When the initially transferred sugar is a GalNAc, the core structures that form differ depending on the type of the second and third sugars and their bonding patterns. There are eight known types of core structures, shown in Fig. 7. The core 1 structure (Galβ1-3GalNAcα1-peptide), also known as the Thomsen-Friedenreich antigen, is expressed *in vivo*, in nearly all tissues and cells. This antigen, along with Tn antigen (GalNAcα1-peptide) and sialyl Tn (STn) antigen (NeuAcα2-6GalNAcα1-peptide), is well-known as a cancer-related sugar-chain antigen in breast cancer, bladder cancer, colon cancer and such. There have been numerous reports that the enzyme activity of core 1 β1,3-galactosyltransferase (an enzyme that transfers galactose to GalNAc of GalNAcα1-peptide through a β1,3 bond), which is required for biosynthesis of this core 1 structure, is for some reason decreased or lost in diseases such as IgA nephropathy and Tn syndrome.

As shown above, abnormalities in galactosyltransferase are associated with a variety of diseases. Thus analyses on galactosyltransferases were anticipated in order to elucidate the causes of these diseases, and to develop treatments. It is hoped that if novel galactosyltransferases can be identified, the relationship between these enzyme and diseases can be better understood.

### Disclosure of the Invention

The present invention takes the above circumstances into account. An objective of the present invention is to identify novel galactosyltransferases.

A further objective of the present invention is to provide uses of the novel galactosyltransferases thus identified. In a preferable embodiment of the novel galactosyltransferases, methods are provided that use enzyme mutations or expression abnormalities as indicators for investigating diseases caused by these enzymes. Moreover, by using enzyme activities as indicators, the present invention provides methods of screening candidate compounds for treating diseases caused by these enzymes.

To solve the aforementioned problems, the present inventors first conducted a search of public databases using known galactosyltransferase C1Gal-T1 as query, and cloned a novel galactosyltransferase from a cDNA library based on the DNA sequences found as a result of the search. A cDNA library of human colon adenoma cell line Colo205, prepared by routine procedures (Yuzuru Ikehara, Hisashi Narimatsu, et al., Glycobiology Vol 9, No. 11, pp. 1213-1224, 1999), was used for samples. A method using typical nucleic acid radioisotope probes was used in the screening procedure.

cDNA clones were prepared by picking up independent plaques that had hybridized with the probe, recovering phages, and then inserting these into pBluescript SK(-) vectors. Next, the cDNA clones were sequenced, revealing an ORF of 957bp, encoding 318 amino acids. The protein encoded by the ORF shares less than 30% homology with the amino acid sequence of C1Gal-T1, which was used as a query. The present inventors named this protein "C1Gal-T2". Amino acid sequence analysis of this protein suggests a typical type 2 membrane protein, seen in nearly all glycosyltransferases.

C1Gal-T2 was also suggested to be a synthetase of core 1 sugar chains (galactose β1-3 acetylgalactosaminyl α1-R), since it showed a strong reaction to pNp-α-GalNAc and did not react to pNp-β-GalNAc during a search for C1Gal-T2 receptor substrates.

The present inventors analyzed bonding patterns between galactose and N-acetylgalactosaminyl α1-R, and as a result also found C1Gal-T2 to be a core 1 synthetase *in vivo.*

In addition, the present inventors analyzed the gene expression of C1Gal-T1 and the discovered C1Gal-T2, using core 1 synthetase-deficient cell lines (LSC and Jurkat). The results showed that core 1 synthesis activity is absent in the LSC and Jurkat cell lines because C1Gal-T2 is in its inactive form. Furthermore, C1Gal-T1 enzyme activity could not be detected despite its mRNA expression, suggesting that C1Gal-T2 is a core 1 synthetase having stronger specific activity.

The present inventors also conducted a database search using the identified C1Gal-T2 as a query, and found a protein approximately 68% homologous at the amino acid sequence level. Until then, only the genome sequence information of this protein-encoding gene was known. The present inventors named this sequence C1Gal-T3, and discovered that it comprises galactosyltransferase activity.

As described above, the present inventors successfully identified novel galactosyltransferases (proteins comprising galactose transferring activity) C1Gal-T2 and C1Gal-T3, thus completing the present invention. Galactosyltransferases are considered to comprise important functions *in vivo,* and abnormalities in their expressions and functions may cause various diseases. Consequently, such diseases can be tested for by using the activities or expressions of the identified galactosyltransferases as indicators. The novel galactosyltransferases identified, and the polynucleotides encoding them, are expected to be suitable therapeutic agents for these diseases.

The present invention relates to novel galactosyltransferases and their genes, to methods for producing them, and to uses of the same, and specifically, relates to:
[1] a polynucleotide selected from the group consisting of:
   (a) a polynucleotide that encodes a polypeptide comprising the amino acid sequence of SEQ ID NO: 2 or 19; and
   (b) a polynucleotide that comprises a coding region of the nucleotide sequence of SEQ ID NO: 1 or 18;
[2] a polynucleotide comprising galactose transferring activity selected from the group consisting of:
   (c) a polynucleotide that encodes a polypeptide comprising the amino acid sequence of SEQ ID NO: 2 or 19 wherein one or more amino acid are substituted, deleted, added, and/or inserted; and
   (d) a polynucleotide that hybridizes with a DNA comprising the nucleotide sequence of SEQ ID NO: 1 or 18 under stringent conditions;
[3] a polynucleotide that encodes a fragment of a polypeptide comprising the amino acid sequence of SEQ ID NO: 2 or 19;
[4] a vector that comprises the polynucleotide of any one of [1] to [3];
[5] a host cell that comprises the polynucleotide of any one of [1] to [3] or the vector of [4];
[6] a polypeptide encoded by the polynucleotide of any one of [1] to [3];
[7] a method for producing the polypeptide of [6], which comprises the steps of:
   culturing the host cell of [5]; and
   recovering the polypeptide produced from the host cell or the culture supernatant of the same;
[8] an antibody that binds to the polypeptide of [6];
[9] a pharmaceutical composition for treating a patient who requires an increase in the activity or expression of the polypeptide of [6], wherein the composition comprises a therapeutically effective amount of a molecule selected from the group consisting of:
   (a) the polynucleotide of any one of [1] to [3];
   (b) the vector of [4]; and
   (c) the polypeptide of [6];
[10] a pharmaceutical composition for treating a patient who requires suppression of the activity or expression of the polypeptide of [6], wherein the composition comprises a therapeutically effective amount of a molecule selected from the group consisting of:
   (a) the antibody of [8]; and
   (b) a polynucleotide that suppresses the expression of an endogenous gene encoding the polypeptide of [6] *in vivo;*
[11] a method of screening for a candidate therapeutic compound for a disease related to abnormal expression of a gene encoding the polypeptide of [6], or abnormal activity of the polypeptide of [6], which comprises the steps of:
   (a) contacting a test compound with the polypeptide of [6];
   (b) measuring the galactose transferring activity of the polypeptide of [6]; and
   (c) selecting a compound that changes the galactose transferring activity, compared to when the test compound is not contacted;
[12] a method of testing for a disease related to abnormal expression of a gene encoding the polypeptide of [6] or abnormal activity of the polypeptide of [6], which comprises the step of detecting a mutation in the gene or its expression control region, in a patient;
[13] the method of [12], which comprises the steps of:
   (a) preparing a DNA sample from a subject;
   (b) isolating a DNA that encodes the polypeptide of [6] or its expression control region;
   (c) determining the nucleotide sequence of the isolated DNA; and
   (d) comparing the nucleotide sequence of the DNA of step (c) with that of a control;
[14] the method of [12], which comprises the steps of:
   (a) preparing a DNA sample from a subject;
   (b) cleaving the prepared DNA sample with a restriction enzyme;
   (c) separating the DNA fragments by size; and
   (d) comparing the size of the detected DNA fragment with that of a control;
[15] the method of [12], which comprises the steps of:
   (a) preparing a DNA sample from a subject;
   (b) amplifying a DNA that encodes the polypeptide of [6] or its expression control region;
   (c) cleaving the amplified DNA with a restriction enzyme;
   (d) separating the DNA fragments by its size; and
   (e) comparing the size of the detected DNA fragments with that of a control;
[16] the method of [12], which comprises the steps of:
   (a) preparing a DNA sample from a subject;
   (b) amplifying a DNA that encodes the polypeptide of [6] or its expression control region;
   (c) dissociating the amplified DNA into a single strand DNA;
   (d) separating the dissociated single strand DNA on non-denaturing gel; and
   (e) comparing the mobility of the separated DNA on the gel with that of a control;
[17] the method of [12], which comprises the steps of:
   (a) preparing a DNA sample from a subject;
   (b) amplifying a DNA that encodes the polypeptide of [6] or its expression control region;
   (c) separating the amplified DNA on a gel that comprises gradually increasing concentration of a DNA denaturant; and
   (d) comparing the mobility of the separated DNA on the gel with that of a control;
[18] a method of testing for a disease related to abnormal expression of a gene encoding the polypeptide of [6], which comprises the step of detecting the expression level of the gene in a subject;
[19] the method of [18], which comprises the steps of:
   (a) preparing an RNA sample from a subject;
   (b) measuring the amount of RNA that encodes the polypeptide of [6] comprised in the RNA sample; and
   (c) comparing the measured amount of RNA with that of a control;
[20] the method of [18], which comprises the steps of:
   (a) providing a cDNA sample prepared from a subject, and a board on which a nucleotide probe that hybridizes with a DNA encoding the polypeptide of [6] is immobilized;
   (b) contacting the cDNA sample with the board;
   (c) measuring the expression level of a gene encoding the polypeptide of [6] comprised in the cDNA sample by detecting the intensity of hybridization between the cDNA sample and the nucleotide probe immobilized on the board; and
   (d) comparing the measured expression level of the gene encoding the polypeptide of [6] with that in a control;
[21] the method of [18], which comprises the steps of:
   (a) preparing a protein sample from a subject;
   (b) measuring the amount of the polypeptide of [6] comprised in the protein sample; and
   (c) comparing the measured amount of the polypeptide with that of a control;
[22] the method of any one of [12] to [21], wherein the disease is IgA nephropathy or Tn syndrome;
[23] an oligonucleotide comprising at least 15 nucleotides that hybridizes with a DNA encoding the polypeptide of claim 6 or an expression control region thereof;
[24] A drug comprising the oligonucleotide of claim 23, for testing for a disease related to abnormal expression of a gene encoding the polypeptide of claim 6, or abnormal activity of the polypeptide of claim 6;
[25] a pharmaceutical comprising the antibody of claim 8, for testing for a disease related to abnormal expression of a gene encoding the polypeptide of claim 6, or abnormal activity of the polypeptide of claim 6;
[26] the pharmaceutical of [24] or [25], wherein the disease is IgA nephropathy or Tn syndrome;
[27] a genetically altered non-human animal wherein the expression of C1Gal-T2 protein is artificially altered;
[28] a genetically altered non-human animal into which an exogenous polynucleotide that encodes C1Gal-T2 protein has been introduced;
[29] the genetically altered non-human animal of [27] or [28], wherein the non-human animal is a mouse;
[30] a cell established from the genetically altered non-human animal of any one of claims 27 to 29; and
[31] a method of screening for a compound that changes the activity of C1Gal-T2 protein, which comprises the steps of:
   (a) administering a test compound to the genetically altered non-human animal of any one of [27] to [29], or contacting the test compound with the cell of [30];
   (b) measuring the activity or expression level of C1Gal-T2 protein in the genetically altered non-human animal or the cell; and
   (c) selecting a compound that changes the activity or expression level of C1Gal-T2 protein by comparison with activity in the absence of the test compound.

The terms used herein are defined below. These terms are described to facilitate understanding of the present description, and it should be understood that they should not be used to limit the present invention.

Herein, "galactosyltransferase" refers to an enzyme that transfers galactose to the hydroxyl groups of a sugar, sphingosine, ceramide, diacylglycerol, hydroxylysine, and such, by using UDP galactose as a donor. The galactosyltransferases of the present invention typically refer to proteins that comprise galactose transferring activity. The phrase, "proteins comprising galactose transferring activity", not only refers to proteins that themselves comprise galactose transferring activity, but also encompasses cases where proteins that do not comprise such an activity themselves interact with other proteins (to form a complex, for example) and these proteins (complex) exhibit galactose transferring activity.

The term "polynucleotide" as used herein refers to a ribonucleotide or deoxyribonucleotide, or a polymer consisting of a number of bases or base pairs. Polynucleotides include single-stranded DNAs as well as double-stranded DNAs. Polynucleotides include both unmodified naturally-occurring polynucleotides and modified polynucleotides. Tritylated bases and special bases such as inosine are examples of modified bases.

The term "polypeptide" as used herein refers to a polymer comprising a number of amino acids. Therefore, oligopeptides and proteins are also included within the concept of polypeptides. Polypeptides include both unmodified naturally occurring polypeptides and modified polypeptides. Examples of polypeptide modifications include acetylation; acylation; ADP-ribosylation; amidation; covalent binding with flavin; covalent binding with heme moieties; covalent binding with nucleotides or nucleotide derivatives; covalent binding with lipids or lipid derivatives; covalent binding with phosphatidylinositols; cross-linkage; cyclization; disulfide bond formation; demethylation; covalent cross linkage formation; cystine formation pyroglutamate formation; formylation; γ-carboxylation; glycosylation; GPI-anchor formation; hydroxylation; iodination; methylation; myristoylation; oxidation; proteolytic treatment; phosphorylation; prenylation; racemization; selenoylation; sulfation; transfer RNA-mediated amino acid addition to a protein such as arginylation; ubiquitination; and the like.

The term "isolate" as used herein refers to a substance (for example, polynucleotide or polypeptide) taken from its original environment (for example, the natural environment for a naturally-occurring substance) and "artificially" changed from its natural state. "Isolated" compounds refer to compounds comprising those present in samples that are substantially abundant with a subject compound, and/or those present in samples wherein the subject compound is partly or substantially purified. Herein, the term "substantially purified" refers to compounds (for example, polynucleotides or polypeptides) that are isolated from the natural environment and in which at least 60%, preferably 75%, and most preferably 90% of the other components associated with the compound in nature are absent.

The term "mutation" as used herein refers to changes to the amino acids of an amino acid sequence, or changes to the bases in a nucleotide sequence (that is, substitution, deletion, addition, or insertion of one or more amino acids or nucleotides). Therefore, the term "mutant" as used herein refers to amino acid sequences wherein one or more amino acids are changed, or nucleotide sequences wherein one or more nucleotides are changed. Nucleotide sequence changes in the mutant may change the amino acid sequence of the polypeptide encoded by the standard polynucleotide, or not. The mutant may be one that exists in nature, such as an allelic mutant, or one not yet identified in nature. The mutant may be conservatively altered, wherein substituted amino acids comprise similar structural or chemical characteristics to the original amino acid. Rarely, mutants may be substituted non-conservatively. Computer programs known in the art, such as DNA STAR software, can be used to decide which or how many amino acid residues to substitute, insert, or delete without inhibiting biological or immunological activities.

"Deletion" is a change to either an amino acid sequence or nucleotide sequence, wherein one or more amino acid residues or nucleotide residues are missing when compared with the amino acid sequence of a naturally occurring galactosyltransferase and galactosyltransferase-associated polypeptide, or a nucleotide sequence encoding the same.

"Insertion" or "addition" is a change to either an amino acid sequence or nucleotide sequence, wherein one or more amino acid residues or nucleotide residues are added compared with the amino acid sequence of a naturally-occurring galactosyltransferase and galactosyltransferase-associated polypeptide, or a nucleotide sequence encoding the same.

"Substitution" is a change to either an amino acid sequence or nucleotide sequence, wherein one or more amino acid residues or nucleotide residues are changed to different amino acid residues or nucleotide residues, compared to the amino acid sequence of a naturally-occurring galactosyltransferase and galactosyltransferase-associated polypeptide, or a nucleotide sequence encoding the same.

The term "hybridize" as used herein refers to a process wherein a nucleic acid chain binds to its complementary chain through the formation of base pairs.

In general, the term "treatment" as used herein means to achieve pharmacological and/or physiological effects. Such effects may be either prophylactic effects completely or partially preventing a disorder or symptom, or therapeutic effects completely or partially curing a symptom of a disorder. The term "treatment" as used herein encompasses all treatments of disorders in mammals, and in particular, in humans. Moreover, this term also includes prophylaxis of the onset of the disease, suppression of disorder progression, and amelioration of a disease in subjects with disease diathesis who have not been diagnosed as being ill.

### <Polypeptides>

The present invention presents novel polypeptides that encode galactosyltransferases. In the present invention, SEQ ID NO: 1 is a nucleotide sequence of the polynucleotide encoding novel galactosyltransferase C1Gal-T2, which was identified by the present inventors. SEQ ID NO: 2 denotes the amino acid sequence of the polypeptide encoded by the polynucleotide. In addition, SEQ ID NO: 18 denotes the nucleotide sequence of a polynucleotide that encodes the novel galactosyltransferase C1Gal-T3, which was identified by the present inventors; SEQ ID NO: 19 denotes the amino acid sequence of the polypeptide encoded by the polynucleotide.

The present invention also provides polypeptides that are functionally equivalent to the polypeptides identified by the present inventors. Herein, the phrase "functionally equivalent" refers to any subject polypeptide that comprises a biological characteristic equivalent to that of a polypeptide identified by the present inventors. The biological characteristics of galactosyltransferases include the activity of transferring galactose to the hydroxyl groups of a sugar, sphingosine, ceramide, diacylglycerol, or hydroxylysine (galactose transferring activity). In a preferable embodiment of the present invention, an example is the enzyme activity of transferring galactose to GalNAc of GalNAcα1-peptide through a β1,3 bond (core 1 β1,3-galactosyltransferase activity).

Thus, whether a subject polypeptide comprises a biological characteristic equivalent to that of a polypeptide identified by the present inventors can be determined by measuring galactose transferring activity, using procedures commonly known to those skilled in the art. For example, an activity can be determined by using radioisotope-labeled UDP-Gal as a sugar donor substrate to measure the amount of radiation taken up by the product. More specifically, galactose transferring activity can be measured by using the methods described in the Examples below.

Methods for introducing mutations into the amino acid sequence of proteins are one embodiment of the methods for preparing polypeptides functionally equivalent to the polypeptides identified by the inventors. Such methods include, for example, site-directed mutagenesis (Current Protocols in Molecular Biology, edit. Ausubel et al. (1987) Publish. John Wiley & Sons Section 8.1-8.5). Amino acid mutation in polypeptides may also occur in nature. The present invention includes mutant proteins, regardless of whether artificially or naturally produced, that comprise the amino acid sequence identified by the inventors (SEQ ID NO: 2 or 19), wherein one or more amino acid residues are altered by substitution, deletion, insertion, and/or addition, yet which are functionally equivalent to the polypeptides identified by the present inventors.

From the viewpoint of conserving the protein's functions, an amino acid residue used for substitution preferably has properties similar to the substituted amino acid residue. For example, Ala, Val, Leu, Ile, Pro, Met, Phe, and Trp are all classified as non-polar amino acids, and are considered to comprise similar properties. Further, examples of uncharged amino acids are Gly, Ser, Thr, Cys, Tyr, Asn, and Gln. Moreover, examples of acidic amino acids are Asp and Glu, and those of basic amino acids are Lys, Arg, and His.

There are no limitations as to the number and site of the amino acid mutations of these polypeptides, so long as the mutated polypeptides retain a function of the original polypeptide. The number of mutations may be typically less than 10%, preferably less than 5%, and more preferably less than 1% of the total amino acid residues.

Other embodiments of the methods for preparing polypeptides functionally equivalent to the polypeptides identified by the inventors include methods utilizing hybridization techniques or gene amplification techniques. More specifically, those skilled in the art can obtain polypeptides functionally equivalent to the polypeptides determined by the present inventors by isolating highly homologous DNAs from DNA samples derived from organisms of the same or different species using hybridization techniques (Current Protocols in Molecular Biology, edit. Ausubel et al. (1987) Publish. John Wiley & Sons Section 6.3-6.4) based on the DNA sequence encoding the polypeptides identified by the inventors (SEQ ID NO: 1 or 18). Thus, such polypeptides encoded by DNAs hybridizing to the DNAs encoding the polypeptides identified by the inventors, which are functionally equivalent to the polypeptides identified by the inventors, are also included in the polypeptides of this invention.

Examples of organisms for use in isolating such polypeptides are rats, mice, rabbits, chicken, pigs, cattle, and such, as well as humans, but the present invention is not limited to these organisms.

Hybridization stringencies required to isolate a DNA encoding a polypeptide functionally equivalent to the polypeptides identified by the inventors are normally "1x SSC, 0.1% SDS, 37°C" or such, more stringent conditions being "0.5x SSC, 0.1% SDS, 42°C" or such, and much more stringent conditions being "0.2x SSC, 0.1% SDS, 65°C" or such. DNAs with higher homology to the probe sequence are expected to be isolated at higher stringencies. However, the above-mentioned combinations of SSC, SDS, and temperature conditions are only examples, and those skilled in the art can achieve the same stringencies as described above by appropriately combining the above-mentioned factors or other parameters which determine hybridization stringency (for example, probe concentration, probe length, reaction time of hybridization, etc.).

The polypeptides encoded by DNAs isolated using such hybridization techniques normally comprise amino acid sequences highly homologous to the polypeptides identified by the present inventors. Herein, high homology indicates sequence identity of at least 40% or more, preferably 60% or more, more preferably 80% or more, still more preferably 90% or more, further still more preferably at least 95% or more, and yet more preferably at least 97% or more (for example, 98% to 99%). Homology of amino acid sequences can be determined, for example, using the algorithm BLAST of Karlin and Altschul (Proc. Natl. Acad. Sci. USA 87: 2264-2268 (1990); Proc. Natl. Acad. Sci. USA 90: 5873-5877 (1993)). Based on this algorithm, a program referred to as BLASTX has been developed (Altschul et al., J. Mol. Biol. 215: 403-410 (1990)). When amino acid sequences are analyzed using BLASTX, parameters are set, for example, at score = 50 and wordlength = 3, while when using BLAST and Gapped BLAST programs, default parameters of each program are used. Specific techniques for these analytical methods are well known in the field (see http://www.ncbi.nlm.nih.gov.).

Gene amplification techniques (PCR) (Current Protocols in Molecular Biology, edit. Ausubel et al. (1987) Publish. John Wiley & Sons Section 6.1-6.4) can be utilized to obtain polypeptides functionally equivalent to the polypeptides isolated by the present inventors, based on DNA fragments isolated as DNAs highly homologous to the DNA sequences encoding the polypeptides isolated by the present inventors. This can be achieved by designing primers based on a part of the DNA sequence encoding the polypeptides identified by the inventors (SEQ ID NO: 1 or 18).

Polypeptides of this invention may be in the form of "mature" proteins, or may also be part of larger proteins, such as fusion proteins. Polypeptides of this invention may comprise secretory sequences, namely leader sequences; prosequences; sequences useful for purification, such as multiple histidine residues and such; and additive sequences to secure stability during recombinant production.

### <Polypeptide fragments>

The present invention also provides fragments of the polypeptides of this invention. These fragments are polypeptides comprising amino acid sequences that are partly, but not entirely, identical to the above polypeptides of this invention. The polypeptide fragments of this invention usually comprise eight amino acid residues or more, and preferably twelve amino acid residues or more (for example, 15 amino acid residues or more). Examples of preferred fragments include truncation polypeptides, comprising amino acid sequences that lack a series of amino acid residues including either the amino terminus or carboxyl terminus, or two series of amino acid residues, one including the amino terminus and the other including the carboxyl terminus. Furthermore, fragments featuring structural or functional characteristics are also preferable, and include those having α-helix and α-helix forming regions, β-sheet and β-sheet forming regions, turn and turn-forming regions, coil and coil-forming regions, hydrophilic regions, hydrophobic regions, α-amphipathic regions, β-amphipathic regions, variable regions, surface forming regions, substrate-binding regions, and high antigenicity index regions. Biologically active fragments are also preferred. Biologically active fragments mediate the activities of the polypeptides of this invention, and include those comprising a similar or improved activity, or a reduced undesirable activity. For example, fragments comprising antigenicity or immunogenicity in animals, especially humans, are included. These polypeptide fragments preferably retain a biological activity, including antigenicity, of the polypeptides of this invention. Mutants of specific sequences or fragments also constitute an aspect of this invention. Preferred mutants are those that differ from the subject polypeptide due to replacement with conservative amino acids, namely, those in which a residue is substituted with another residue of similar properties. Typical substitutions are those between Ala, Val, Leu, and Ile; Ser and Thr; acidic residues Asp and Glu, Asn, and Gln; basic residues Lys and Arg; or aromatic residues Phe and Tyr.

### <Production of polypeptides>

Polypeptides of this invention may be produced by any appropriate method. Such polypeptides include isolated naturally-occurring polypeptides, and polypeptides which are produced by gene recombination, synthesis, or by a combination thereof. Procedures for producing these polypeptides are well known in the art. Recombinant polypeptides may be prepared, for example, by transferring a vector, inserted with a polynucleotide of the present invention, into an appropriate host cell, and purifying the polypeptide expressed within the resulting transformant. On the other hand, naturally occurring polypeptides can be prepared, for example, using affinity columns wherein antibodies against a polypeptide of this invention (described below) are immobilized (Current Protocols in Molecular Biology, edit. Ausubel et al. (1987) Publish. John Wiley & Sons, Section 16.1-16.19). Antibodies for affinity purification may be either polyclonal or monoclonal antibodies. The polypeptides of this invention may be also prepared by in vitro translation methods (for example, see "On the fidelity of mRNA translation in the nuclease-treated rabbit reticulocyte lysate system." Dasso, M. C. and Jackson, R. J. (1989) NAR 17: 3129-3144), and such. The polypeptide fragments of this invention can be produced, for example, by cleaving the polypeptides of the present invention with appropriate peptidases.

### <Polynucleotides>

The present invention also provides polynucleotides encoding the polypeptides of this invention. The polynucleotides of this invention include those encoding polypeptides comprising the amino acid sequence of SEQ ID NO: 2 or 19; those comprising coding regions of the nucleotide sequence of SEQ ID NO: 1 or 18; and those comprising nucleotide sequence different from that of SEQ ID NO: 1 or 18 due to genetic code degeneracy, but still encoding polypeptides comprising the amino acid sequence of SEQ ID NO: 2 or 19. Furthermore, the polynucleotides of this invention include those encoding polypeptides functionally equivalent to the polypeptides of the present invention, comprising nucleotide sequences which are at least 40% or more homologous to the said polynucleotide sequences, preferably 60% or more, more preferably 80% or more, further more preferably 90% or more, still preferably 95% or more, and further still more preferably 97% or more (for example, 98% to 99%) over the entire length. Homology of nucleotide sequences can be determined, for example, using the BLAST algorithm by Karlin and Altschul (Proc. Natl. Acad. Sci. USA 87: 2264-2268 (1990); Proc. Natl. Acad. Sci. USA 90: 5873-5877 (1993)). Based on this algorithm, an algorithm called BLASTN has been developed (Altschul et al. J. Mol. Biol. 215: 403-410 (1990)). When analyzing a nucleotide sequence using the BLASTN program, the parameters are set, for example, at score = 100 and wordlength = 12. When using both BLAST and Gapped BLAST programs, default parameters for each program are used. The specific techniques for these analytical methods are well known in the art (http://www.ncbi.nlm.nih.gov.). The polynucleotides of this invention also include polynucleotides comprising nucleotide sequences complementary to those of the above-described polynucleotides.

The polynucleotides of this invention can be obtained by standard cloning and screening methods, for example, from cDNA libraries induced from intracellular mRNAs. Moreover, the polynucleotides of this invention can be obtained from natural sources, such as genomic libraries, and also can be synthesized using commercially available techniques known in the art.

Polynucleotides comprising nucleotide sequences significantly homologous to the polynucleotide sequence identified by the inventors (SEQ ID NO: 1 or 18) can be prepared using, for example, hybridization techniques (Current Protocols in Molecular Biology, edit. Ausubel et al. (1987) Publish. John Wiley & Sons Section 6.3-6.4) and gene amplification techniques (PCR) (Current Protocols in Molecular Biology, edit. Ausubel et al. (1987) Publish. John Wiley & Sons Section 6.1-6.4). That is, based on the polynucleotide sequence identified by the present inventors (SEQ ID NO: 1 or 18) or portions thereof, hybridization techniques can be used to isolate DNAs highly homologous to these polynucleotides, from DNA samples derived from the same or different species of organisms. Moreover, polynucleotides highly homologous to a sequence of a said polynucleotide can be isolated using gene amplification techniques, by designing primers based on portions of the polynucleotide sequence identified by the present inventors (SEQ ID NO: 1 or 18). Therefore, the present invention includes polynucleotides that hybridize under stringent conditions to polynucleotides comprising the nucleotide sequence of SEQ ID NO: 1 or 18. Conditions for stringent hybridization are usually "1x SSC, 0.1% SDS, 37°C" or such, with more stringent conditions being "0.5x SSC, 0.1% SDS, 42°C" or such, and further more stringent conditions being "0.2x SSC, 0.1% SDS, 65°C" or such. The more stringent the hybridization conditions, the greater the homology of the isolated DNAs to the probe sequence. However, the above-described combinations of SSC, SDS, and temperature conditions are mere examples, and those skilled in the art can achieve stringencies similar to the above-described by appropriately combining the aforementioned factors or other parameters that determine hybridization stringency (for example, probe concentration, probe length, reaction time of hybridization, etc.).

Polynucleotides comprising nucleotide sequences significantly homologous to the sequences of the polyncleotides identified by the inventors can also be prepared by methods of introducing mutations into the nucleotide sequence of SEQ ID NO: 1 or 18 (for example, site-directed mutagenesis) (Current Protocols in Molecular Biology, edit. Ausubel, et al. (1987) Publish. John Wiley & Sons Section 8.1-8.5). Such polynucleotides may be also generated by natural mutations. The present invention includes polynucleotides encoding polypeptides comprising the amino acid sequence of SEQ ID NO: 2 or 19, wherein one or more amino acid residues are substituted, deleted, inserted, and/or added by these nucleotide sequence mutations.

Polynucleotides used for the recombinant production of the polypeptides of this invention include the coding sequences of mature polypeptides or fragments thereof alone; and coding sequences of mature polypeptides or fragments thereof in the same reading frame with other coding sequences (for example, leader or secretory sequences; pre-, pro-, or preproprotein sequences; or sequences encoding other fusion peptide portions). For example, a marker sequence that facilitates purification of the fusion polypeptide may be encoded in the same reading frame. A preferred embodiment of this invention includes specific marker sequences, such as the hexahistidine peptide or Myc tag provided by the pcDNA3.1/Myc-His vector (Invitrogen), which is described in the literature (Gentz et al., Proc. Natl. Acad. Sci. USA (1989) 86: 821-824). Further, this polynucleotide may comprise 5'- and 3'-noncoding sequences, for example, transcribed but non-translated sequences; splicing and polyadenylation signals; ribosome-binding sites; and mRNA stabilization sequences.

### <Probes, primers, antisenses, and ribozymes>

The present invention provides nucleotides with a chain length of at least 15 nucleotides, which are complementary to a polynucleotide isolated by the present inventors (a polynucleotide or a complementary strand thereof comprising the nucleotide sequence of SEQ ID NO: 1 or 18). Herein, the term "complementary strand" is defined as the other strand of a double-stranded nucleic acid composed of A:T (A:U in case of RNA) and G:C base pairs. Also, "complementary" is defined as not only complete matching within a continuous region of at least 15 sequential nucleotides, but also homology of at least 70%, preferably at least 80%, more preferably 90%, and most preferably 95% or higher within that region. Homology may be determined using an algorithm described herein. Probes and primers for detection or amplification of the polynucleotides of the present invention are included in these polynucleotides. Typical polynucleotides used as primers are 15 to 100 nucleotides long, and preferably 15 to 35 nucleotides long. Alternatively, polynucleotides used as probes are nucleotides at least 15 nucleotides in length, and preferably at least 30 nucleotides. They comprise at least a portion or an entire sequence of a DNA of the present invention. Such nucleotides preferably hybridize specifically to a DNA encoding a polypeptide of the present invention. The term "hybridize specifically" refers to hybridization under normal hybridization conditions, preferably stringent conditions, with the nucleotide identified by the present inventors (SEQ ID NO: 1 or 18), but not with DNAs encoding other polypeptides.

These nucleotides can be used for detecting and diagnosing an abnormal activity of the polypeptides of the present invention, or abnormal expression of genes encoding these polypeptides.

Further, these nucleotides include polynucleotides that suppress the expression of genes encoding the polypeptides of the present invention. Such polynucleotides include antisense polynucleotides (antisense DNA/RNA; antisense RNAs which are complementary to the transcription products of the genes encoding the polypeptides of the present invention, and DNAs encoding the RNAs) and ribozymes (DNAs encoding RNAs that comprise a ribozyme activity of specifically cleaving transcriptional products of the genes encoding the polypeptides of the present invention).

A number of factors, such as those described below, cause the mechanisms of antisense polynucleotide actions that suppress the expression of a target gene: inhibition of transcription initiation by the formation of a triple strand; suppression of transcription through hybridization with a local open loop conformation site formed by an RNA polymerase; inhibition of transcription by hybridization with RNA that is being synthesized; suppression of splicing through hybridization at an intron-exon junction; suppression of splicing through hybridization with a spliceosome forming site; suppression of transfer from the nuclei to cytoplasm through hybridization with mRNA; suppression of splicing through hybridization with a capping site or poly(A) addition site; suppression of translation initiation through hybridization with a translation initiation factor binding site; suppression of translation through hybridization with a ribosome binding site near the initiation codon; inhibition of peptide chain elongation through hybridization with the translation regions and polysome binding sites of mRNAs; suppression of gene expression by hybridization with the interaction sites between nucleic acids and proteins; and the like. These actions inhibit the processes of transcription, splicing, and/or translation, suppressing the expression of a target gene (Hirajima and Inoue, "New Biochemistry Experimental Course No. 2, Nucleic Acid IV, Duplication and Expression of Genes", Japan Biochemical Society ed., Tokyo Kagaku Doujin, pp. 319-347 (1993)).

The antisense polynucleotides of the present invention may suppress target gene expression through any of the above-mentioned actions. According to one embodiment, an antisense sequence designed to be complementary to a non-translated region near the 5'-terminus of mRNA of a gene may effectively inhibit the translation of that gene. Additionally, sequences which are complementary to a coding region or a 3' non-translated region can be also used. As described above, polynucleotides comprising sequences antisense not only to the translated region of a gene, but also to a non-translated region are included in the antisense polynucleotides of the present invention. The antisense polynucleotides to be used in the present invention are linked downstream of an appropriate promoter, and a sequence including a transcriptional termination signal is preferably linked to the 3'-side thereof. The sequence of an antisense polynucleotide is preferably complementary to the target gene or a part thereof; however, so long as gene expression can be effectively inhibited, complete complementarity is unnecessary. A transcribed RNA is preferably 90% or more complementary to the transcribed product of the target gene, and more preferably 95% or more. In order to effectively inhibit the expression of a target gene using an antisense sequence, the antisense polynucleotide comprises at least 15 or more, preferably 100, more preferably 500 nucleotides, and usually comprises less than 3000, preferably less than 2000 nucleotides, to cause an antisense effect.

This type of antisense polynucleotide may also be applied to gene therapies for diseases caused by abnormalities in the polypeptides of the present invention (abnormalities of function or expression).

Sialyl Lewis X sugar chains and sialyl 6-sulfo Lewis X sugar chains are ligands of selectin adhesion molecules, and are said to be involved in the homing phenomenon of lymphocytes, adhesions to vascular endothelial cells for leucocyte extravasation at local inflammatory sites, and hematogenic metastasis of cancer cells. These sugar chain antigens are present in both glycoproteins and glycolipids, as well as in both N-linked and O-linked chains of glycoproteins. In O-linked sugar chains, the sugar chain elongates mainly from the core 1 structure, and branches off, with sialyl Lewis X and sialyl 6-sulfo Lewis X sugar chains on the non-reducing side. Accordingly, the expression of these sugar chain antigens may be suppressed by inhibiting synthesis of the core 1 structure. More specifically, anticancer functions or anti-inflammatory effects may be expected on suppressing the activity or expression of core 1 synthetase (C1Gal-T). For example, an antisense polynucleotide of C1Gal-T2 or C1Gal-T3 of the present invention may be used as an enzyme activity inhibitor to suppress anti-inflammatory action and hematogenic metastasis.

The antisense polynucleotides can be prepared by, for example, phosphorothionate methods (Stein, "Physicochemical properties of phosphorothioate oligodeoxynucleotides." Nucleic Acids Res. 16, 3209-21 (1988)) and the like, based on the sequence information of a polynucleotide encoding the polypeptide of the present invention (for example, SEQ ID NO: 1 or 18).

Further, suppression of endogenous gene expression can also be achieved by utilizing polynucleotides that encode ribozymes. Ribozymes are RNA molecules with catalytic activity. Ribozymes comprising various activities exist, and research into ribozymes as enzymes for truncating RNA has allowed the design of ribozymes that cleave RNAs in a site-specific manner. There are ribozymes which are larger than 400 nucleotides, such as Group I intron type ribozymes, and M1RNA comprised in RNaseP; and ribosomes which comprise an active domain of about 40 nucleotides, called hammerhead-type and hairpin-type ribozymes (Makoto Koizumi and Eiko Ohtsuka, (1990), Protein Nucleic Acid and Enzyme (PNE) 35:2191).

For example, the hammerhead-type ribozyme cleaves the 3'-side of C15 of G13U14C15 within its own sequence. The formation of a base pair between U14 and the A at position nine is important for this activity, and cleavage has been shown to proceed even if the C at position 15 is an A or a U (M. Koizumi et al., (1988) FEBS Lett. 228:225). Restriction enzymatic RNA-truncating ribozymes that recognize UC, UU, and UA sequences in target RNAs may be generated by designing the substrate binding site of the ribozyme to be complementary to the RNA sequence near the target site (M. Koizumi, et al., (1988) FEBS Lett. 239:285; Makoto Koizumi and Eiko Ohtsuka, (1990), Protein Nucleic Acid and Enzyme (PNE) 35:2191); and M. Koizumi et al. (1989), Nucleic Acids Res. 17:7059).

Further, hairpin-type ribozymes are also useful in the context of the present invention. Hairpin-type ribozymes are found on, for example, the minus strand of satellite RNA of tobacco ringspot virus (J. M. Buzayan, Nature 323:349 (1986)). Ribozymes can also be designed to cause target-specific RNA truncations (Y. Kikuchi and N. Sasaki, (1992) Nucleic Acids Res. 19:6751; and Y. Kikuchi, (1992) Chemistry and Organism 30:112).

When the polynucleotides suppressing the expression of the genes encoding the polypeptides of the present invention are used in gene therapy, they may be administered to a patient by *ex vivo* or *in vivo* methods and the like, using, for example, viral vectors such as retroviral vectors, adenoviral vectors, adeno-associated viral vectors, and such; and non-viral vectors such as liposomes; and such.

A novel glycooligopeptide GSP-6 that binds to the adhesion molecule P-selectin, which appears on the surface of platelets accompanying inflammation, was also discovered by a recent study (Leppanen, A. et al., J. Biol. Chem. 274, 24838-24848, 1999). GSP-6 inhibits lymphocytes from contacting with P-selectin. Thus, GSP-6 is considered to comprise an inhibitory function in inflammatory responses involving selectin, hematogenic metastasis of cancer cells, and such. GSP-6 can be a useful molecule for developing anti-inflammatory agents and anticancer agents. Since core 1 sugar chain synthetase is required to synthesize GSP-6, the polypeptides of the present invention are extremely valuable considering their use in synthesizing glycooligopeptide GSP-6, which inhibits cell adhesion.

Endogenous gene expression can also be inhibited by RNA interference (RNAi), which uses double-stranded RNAs comprising sequences identical or similar to a target gene sequence. RNAi refers to a phenomenon in which the expression of both the introduced exogenous gene and the target endogenous gene are inhibited upon introducing cells with a double-stranded RNA comprising a sequence identical or similar to the target gene sequence. Although the details of the RNAi mechanism are not fully understood, the double-stranded RNAs initially introduced are thought to be degraded into small fragments, and to somehow serve as indicators of the target gene, thereby resulting in target gene degradation. The RNAs used in RNAi are not necessarily, but preferably, perfectly identical to a gene encoding a polypeptide of the present invention, or a partial region of such a gene. In addition, DNA molecules capable of synthesizing double-stranded RNAs intracellularly may also be introduced.

### <Production of vectors, host cells, and polypeptides>

The present invention also provides methods for producing vectors comprising polynucleotides of the present invention, host cells retaining the polynucleotides or said vectors of the present invention, and polypeptides of the present invention utilizing said host cells.

The vectors of the present invention are not limited, so long as the DNAs inserted in the vectors are stably retained. For example, pBluescript vector (Stratagene) is a preferable cloning vector when using *E. coli* as a host. When using vectors to produce the polypeptides of the present invention, expression vectors are particularly useful. These expression vectors are not specifically limited, so long as they express polypeptides *in vitro,* in *E. coli*, in cultured cells, or *in vivo*. However, preferable examples include the pBEST vector (ProMega) for *in vitro* expression, the pET vector (Invitrogen) for expression in *E. coli*, the pME18S-FL3 vector (GenBank Accession No. AB009864) for expression in cultured cells, and the pME18S vector (Mol. Cell Biol. 8:466-472(1988)) for *in vitro* expression, and such. A DNA of the present invention can be inserted into a vector by conventional methods, for example, by a ligase reaction using restriction enzyme sites (Current Protocols in Molecular Biology, edit. Ausubel, et al., (1987) Publish. John Wiley & Sons, Section 11.4-11.11).

Host cells to which the vectors of the present invention are introduced are not specifically limited, and various host cells can be used according to the objectives of the present invention. For example, bacterial cells (e.g. *Streptococcus, Staphylococcus, E. coli, Streptomyces, Bacillus subtilis*), fungal cells (e.g. yeast, Aspergillus), insect cells (e.g. Drosophila S2, Spodoptera SF9), animal cells (e.g. CHO, COS, HeLa, C127, 3T3, BHK, HEK293, Bowes melanoma cell), and plant cells are examples of cells for expressing polypeptides. The transfection of a vector to a host cell can be carried out by conventional methods, such as calcium phosphate precipitation methods, electroporation methods (Current protocols in Molecular Biology, edit., Ausubel et al., (1987) Publish. John Wiley & Sons, Section 9.1-9.9), Lipofectamine methods (GIBCO-BRL), microinjection methods, and such.

In host cells, appropriate secretion signals can be incorporated into a polypeptide of interest in order to secrete an expressed polypeptide into the lumen of the endoplasmic reticulum, into the cavity around a cell, or into the extracellular environment. These signals may be endogenous signals or signals from a species different to the target polypeptide.

When a polypeptide of the present invention is secreted into culture media, this culture media is collected to collect the polypeptide of the present invention. When a polypeptide of the present invention is produced intracellularly, the cells are first lysed, and the polypeptide is then collected.

In order to collect and purify a polypeptide of the present invention from a recombinant cell culture, methods known in the art can be used, including ammonium sulfate or ethanol precipitation, acid extraction, anionic or cationic exchange chromatography, phosphocellulose chromatography, hydrophobic interaction chromatography, affinity chromatography, hydroxylapatite chromatography, and lectin chromatography.

### <Testing methods>

The present invention provides methods for testing for diseases related to abnormal expression of the genes encoding the polypeptides of the present invention, or abnormal activity of the polypeptides of the present invention. Galactosyltransferase is considered to comprise important functions *in vivo*, and thus, its abnormal expression and function may cause various diseases. Therefore, this testing for diseases may be accomplished by using inappropriate activity or expression of the polypeptides of the present invention as an index.

The term "testing for diseases" includes not only testing to plan therapeutic strategies for subjects who exhibit a disease symptom, but also testing to prevent diseases by determining whether a subject is susceptible to the disease or whether a subject already has the disease.

Many recent studies report that galactose transferring activity is decreased or eliminated in diseases such as IgA nephropathy and Tn syndrome. Thus, it is quite likely that abnormal expression of a gene that encodes a polypeptide of the present invention, or an abnormal activity of a polypeptide of the present invention, causes diseases such as IgA nephropathy and Tn syndrome. In the present invention, the phrase "diseases related to abnormal expression of a gene that encodes a polypeptide of the present invention or an abnormal activity of a polypeptide of the present invention" refers to, for example, IgA nephropathy, Tn syndrome.

One embodiment of the testing methods of the present invention is a method that comprises the step of detecting in a subject a mutation in a gene encoding a polypeptide of the present invention, or in an expression control region of such a gene.

In one method, the tests can be accomplished by directly determining the nucleotide sequence of a gene encoding a polypeptide of the present invention, or its expression control region, in a subject. In such methods, a DNA sample is first prepared from a subject. DNA samples can be prepared from chromosomal DNAs or RNAs extracted from the cells of a subject, for example, blood, urine, saliva, and tissue biopsy or autopsy specimens. In order to prepare DNA samples for the present methods from chromosomal DNAs, genomic libraries may be produced by, for example, digesting chromosomal DNAs with appropriate restriction enzymes, and then cloning the digested DNAs in to vectors. When preparing DNA samples of the present method from RNAs, cDNA libraries may be prepared from RNAs using reverse transcriptase. Next, in the present method, DNAs comprising genes encoding polypeptides of the present invention, or their expression control regions, are isolated. DNAs can be isolated by screening genomic or cDNA libraries, using probes that hybridize with DNAs comprising genes encoding the polypeptides of the present invention, or their expression control regions. DNAs can also be isolated by PCR using a genomic DNA library, cDNA library, or RNA as the template, and using primers that hybridize to a DNA comprising a gene encoding a polypeptide, or its expression control region, of the present invention. In the present methods, the nucleotide sequences of the isolated DNAs are then determined. The nucleotide sequences of the selected DNAs can be determined by methods known to those skilled in the art. In the present methods, a DNA's determined nucleotide sequence is compared with that of a control. A "control" herein refers to a nucleotide sequence of a DNA comprising a gene encoding a normal (wild type) polypeptide of the present invention, or its expression control region. When the above comparison shows that the nucleotide sequence of a subject's DNA differs from that of a control, the subject is judged to be afflicted with a disease, or in danger of disease onset.

In the test methods of the present invention, various methods can be used other than the above-described methods of directly determining the nucleotide sequences of subject-derived DNAs.

In one embodiment of the methods, DNA samples are first prepared from subjects, and then digested with restriction enzymes. The DNA fragments are then separated by size, and the detected DNA fragment sizes are compared with those of a control. Alternatively, in another embodiment, DNA samples are first prepared from subjects, then DNAs comprising genes encoding polypeptides of the present invention, or their expression control regions, are amplified from the sample. The amplified DNAs are digested with restriction enzymes, DNA fragments are separated by size, and the sizes of detected DNA fragments are compared with those of a control.

Such methods include, for example, methods utilizing Restriction Fragment Length Polymorphisms/RFLPs, PCR-RFLP methods, and the like. Specifically, when a mutation exists in a restriction enzyme recognition site, or when a DNA fragment generated by restriction enzyme treatment comprises the insertion or deletion of a base, the sizes of fragments generated by restriction enzyme treatment vary compared to those of a control. Portions comprising mutations are amplified by PCR, and are then treated with several restriction enzymes to detect the mutations after electrophoresis, as differences in band mobility. Alternatively, the presence or absence of mutations can be detected by Southern blotting with a probe DNA of the present invention, after treating the chromosomal DNA with various restriction enzymes and carrying out electrophoresis. The restriction enzymes to be used can be appropriately selected according to the mutations. In addition to genomic DNAs, Southern blotting can also be conducted on cDNAs directly digested with restriction enzymes, wherein a reverse transcriptase has converted the RNAs prepared from subjects into cDNAs. Alternatively, after using PCR with a cDNA template to amplify DNAs comprising a gene encoding a polypeptide of the present invention, or its expression control region, the cDNAs are digested with restriction enzymes, and differences in the electrophoresis gel mobility of the DNA fragments generated by the digestion are examined.

In another embodiment of the present methods, DNA samples are first prepared from subjects. Then, DNAs comprising genes encoding polypeptides of the present invention, or their expression control regions, are amplified. The amplified DNAs are then dissociated into single-stranded DNAs, and these single-stranded DNAs are separated on non-denaturing gels. The mobility of the separated single-stranded DNAs on a gel is compared with the mobility of a control.

Such methods include, for example, PCR-SSCP (single-strand conformation polymorphism) methods ("Cloning and polymerase chain reaction-single-strand conformation polymorphism analysis of anonymous Alu repeats on chromosome 11." Genomics. 1992, Jan. 1, 12(1): 139-146; "Detection of p53 gene mutations in human brain tumors by single-strand conformation polymorphism analysis of polymerase chain reaction products." Oncogene. 1991, Aug. 1; 6(8): 1313-1318). These methods are particularly preferable for screening many DNA samples, comprising advantages such as relative ease of operation, the use of only a small amount of test sample, and so on. The principle of such methods is as follows: Single-stranded DNAs, which have been dissociated from double-stranded DNA fragments, form unique conformations that depend on their nucleotide sequences. When these dissociated DNA strands are electrophoresed on a polyacrylamide gel without a denaturant, complementary single-stranded DNAs of the same chain length will shift to different positions, according to differences in their conformations. Even the substitution of a single base changes the conformation of a single-stranded DNA, and such changes result in different mobility during polyacrylamide gel electrophoresis. Accordingly, the presence of a mutation in a DNA fragment, due to a point mutation, deletion, insertion, or so on, can be detected by detecting changes in this mobility.

More specifically, DNAs comprising genes encoding polypeptides of the present invention (or their expression control regions) are first amplified by PCR or the like. Preferably, DNAs of about 200 bp to 400 bp in length are amplified. Those skilled in the art can appropriately select PCR conditions and such. DNA products amplified by PCR can be labeled with primers, which are labeled with isotopes such as ³²P; fluorescent dyes; biotin; and such. Alternatively, the amplified DNA products can be also labeled by conducting PCR in a reaction solution comprising substrate bases, which are labeled with isotopes such as ³²P; fluorescent dyes; biotin; and such. Further, labeling can be also carried out after the PCR reaction by adding substrate bases, which are labeled with isotopes such as ³²P; fluorescent dyes; biotin; and such, to an amplified DNA fragment using Klenow enzyme and such. Then, the obtained labeled DNA fragments are denatured by heating and the like, and electrophoresis is carried out on a polyacrylamide gel, without denaturants such as urea. The conditions for separating DNA fragments by this electrophoresis can be improved by adding appropriate amounts (about 5% to 10%) of glycerol to the polyacrylamide gel. Furthermore, although conditions vary depending on the properties of respective DNA fragments, electrophoresis is usually carried out at room temperature (20°C to 25°C). When preferable separation is not achieved at such a temperature, a temperature at which optimum mobility can be achieved is found between 4°C and 30°C. After electrophoresis, results are analyzed by detecting DNA fragment mobility using autoradiography with X-ray films, scanners for detecting fluorescence, and the like. If a band with different mobility is detected, the presence of a mutation can be confirmed by directly excising the band from the gel, re-amplifying it using PCR, and directly sequencing the amplified fragment. Further, bands can be also detected without using labeled DNAs, by staining the gel after electrophoresis with ethidium bromide, silver, and such.

In still another method, DNA samples are first prepared from a subject, DNAs comprising genes encoding polypeptides of the present invention, or their expression control regions, are amplified, and the amplified DNAs are then separated on a gel comprising a gradient concentration of a DNA denaturant. The gel mobility of the separated DNAs is compared with the mobility of a control.

Denaturant gradient gel electrophoresis methods (DGGE method) and the like are examples of such methods. DGGE methods comprise the steps of: (1) electrophoresing the mixture of DNA fragments on a polyacrylamide gel with a gradient concentration of denaturant; and (2) separating the DNA fragments according to differences in the instability of each fragment. On reaching a gel section with a certain denaturant concentration, unstable DNA fragments comprising mismatches will partly dissociate to a single-strand near these mismatches, due to DNA sequence instability. The mobility of these partly dissociated DNA fragments becomes remarkably slow, resulting in differences in their mobility compared to perfectly double-stranded DNAs without dissociated parts, thus allowing separation of these DNAs. Specifically, DNAs comprising genes encoding polypeptides of the present invention, or their expression control regions, are (1) amplified by PCR and the like with a primer of the present invention or such; (2) electrophoresed on a polyacrylamide gel with a gradient concentration of a denaturant such as urea; and (3) compared with a control using electrophoresis results. The presence or absence of a mutation can be detected by detecting differences in DNA fragment mobility due to the extreme reduction in the mobility speed due to the separation of mutated DNA fragments into single-stranded DNAs at parts of the gel with lower denaturant concentrations.

In addition to the above-mentioned methods, Allele Specific Oligonucleotide (ASO) hybridization methods can be used to detect mutations at only specific sites. An oligonucleotide with a nucleotide sequences comprising a mutation is prepared, and is hybridized with a DNA sample. The existence of a mutation reduces the efficiency of hybridization. This reduction can be detected by Southern blotting; methods using a specific fluorescent reagent that comprises the characteristic of quenching by intercalation into unhybridized gaps; and the like. Further, this can also be detected by ribonuclease A mismatch truncation methods. Specifically, a DNA comprising a gene encoding a polypeptide of the present invention is amplified by PCR or the like. The amplified DNAs are hybridized with labeled RNAs, which were prepared from control cDNAs incorporated into a plasmid vectors, or the like. Those sites that form a single-stranded conformation due to the existence of a mutation are cleaved with ribonuclease A, and the presence of a mutation can thus be detected using autoradiography and the like.

Another embodiment of the test methods of the present invention is a method comprising the step of detecting the expression level of a gene encoding a polypeptide of the present invention. Herein, transcription and translation are included in the meaning of the term "expression of a gene". Accordingly, mRNAs and proteins are included in the term "expression product".

In a method for testing the transcription level of a gene encoding a polypeptide of the present invention, an RNA sample is first prepared from a subject. Then, the amount of RNA that encodes the polypeptide of the present invention in the RNA sample is measured. Thereafter, the measured amount of the RNA encoding the polypeptide of the invention is compared with that of a control.

Examples of such methods include Northern blotting using a probe which hybridizes with a polynucleotide encoding a polypeptide of the present invention; RT-PCR using a primer which hybridizes with a polynucleotide encoding a polypeptide of the present invention; and such.

Furthermore, DNA arrays (Masami Muramatsu and Masashi Yamamoto, New Genetic Engineering Handbook pp. 280-284, YODOSHA Co., LTD.) can also be used in testing the transcription level of genes encoding polypeptides of the present invention. Specifically, a cDNA sample prepared from a subject, and a basal plate on which polynucleotide probes that hybridize with the polynucleotides encoding the polypeptides of the present invention are fixed, are first provided. Several kinds of polynucleotide probes can be fixed on the basal plate in order to detect a plurality of polynucleotides that encode the polypeptides of the present invention. cDNA samples from subjects can be prepared by methods well known to those skilled in the art. In a preferable embodiment of the preparation of a cDNA sample, total RNAs are first extracted from a cell of a subject. Example of cells include blood, urine, saliva, and tissue cells from biopsy or autopsy specimens, and the like. Total RNAs can be extracted as below, for example. Known methods, kits, and such can be used, so long as the total RNAs can be prepared with high purity. For example, total RNAs are pretreated with "RNAlater" (Ambion) and then extracted using "Isogen" (Nippon Gene). The specific procedures of the methods may be carried out according to the attached protocol. cDNA samples are then prepared by synthesizing cDNAs with reverse transcriptase, using extracted total RNAs as a template. cDNAs can be synthesized from total RNAs by conventional methods known in the art. The prepared cDNA samples are labeled for detection, as necessary. There are no specific limits as to the labeling substance, so long as it can be detected. Labeling substances include, for example, fluorescent substances, radioactive elements, and such. Labeling can be carried out by conventional methods (L. Luo et al., "Gene expression profiles of laser-captured adjacent neuronal subtypes", Nat. Med. (1999) pp. 117-122).

Herein, the term "basal plate" refers to a board-type material on which polynucleotides can be fixed. So long as polynucleotides can be immobilized on the plate, the basal plates of the present invention are not restricted. However, basal plates generally used in DNA array techniques are preferable.

One advantage of DNA array techniques is that the amount of solution required for hybridization is very small, and that extremely complicated targets, comprising cDNAs derived from the total RNAs of a cell, can be hybridized to the fixed nucleotide probes. A DNA array generally comprises thousands of nucleotides, printed on to a basal plate at a high density. Usually, DNAs are printed on the surface layer of a non-porous basal plate. The surface layer of the basal plate is usually glass, but a porous film, for example, such as nitrocellulose membrane, can be also used. There are two types of nucleotide fixation (array): one is an array based on polynucleotides developed by Affymetrix Co., Ltd.; and the other is an array of cDNAs mainly developed by Stanford University. Polynucleotides are usually synthesized in situ for polynucleotide arrays. For example, in situ polynucleotide synthesis methods such as photolithographic techniques (Affymetrix), ink-jet techniques (Rosetta Inpharmatics) for fixing chemical substances, and such are already known in the art, and any of these techniques can be used to produce the basal plates of the present invention. The polynucleotide probes to be fixed on the basal plates are not limited, so long as they specifically hybridize with a gene encoding a polypeptide of the present invention. The polynucleotide probes of the present invention include polynucleotides and cDNAs. Herein, the term "specifically hybridizes" means that a polynucleotide substantially hybridizes with a polynucleotide encoding a polypeptide of the present invention, and does not substantially hybridize with other polynucleotides. So long as specific hybridization is possible, polynucleotide probes do not have to be completely complementary to the nucleotide sequences to be detected. Generally, to immobilize a cDNA on to a plate, the length of the polynucleotide probe to be fixed on the basal plate is usually 100 to 4000 bases, preferably 200 to 4000 bases, and more preferably 500 to 4000 bases. On the other hand, to immobilize synthetic polynucleotides, probe length is usually 15 to 500 bases, preferably 30 to 200 bases, and more preferably 50 to 200 bases. Generally, the step of fixing polynucleotides on to a basal plate is also called "printing". Specifically, printing can be conducted, for example, as follows, but is not limited thereto. Several kinds of polynucleotide probes are printed within an area of 4.5 mm x 4.5 mm. In this step, various arrays can be printed using one pin. Thus, when a tool with 48 pins is used, 48 arrays can be repeatedly printed on one standard microscope slide.

The cDNA samples are then contacted with a basal plate of the present method. In this step, the cDNA samples are hybridized on the basal plate with nucleotide probes, which can specifically hybridize with a DNA encoding a polypeptide of the present invention. Although reaction solutions and hybridization reaction conditions vary depending on various factors, such as the length of the nucleotide probe fixed on the basal plate, they can be determined by usual methods known to those skilled in the art.

Next, the expression level of the gene encoding the polypeptide of the present invention comprised in the cDNA sample is measured, by detecting the degree of hybridization of the cDNA sample with the nucleotide probe fixed on the basal plate. The measured expression level of the gene encoding the polypeptide of the present invention is then compared with that of a control.

If a cDNA derived from a gene encoding a polypeptide of the present invention exists in the cDNA sample, that cDNA will hybridize with the nucleotide probe fixed on the basal plate. Thus, the expression level of the gene encoding the polypeptide of the present invention can be measured by detecting the intensity of hybridization of the polynucleotide probe with the cDNA. One skilled in the art can appropriately detect the hybridization intensity of a cDNA with a polynucleotide probe, depending on the kind of substance used to label the cDNA sample. For example, when the cDNAs are labeled with a fluorescent substance, they can be detected by reading the fluorescent signal with a scanner.

In a method of the present invention, the expression level of a gene encoding a polypeptide of the present invention can be measured simultaneously in cDNA samples derived from a subject and control (normal healthy subject), by labeling these samples with different fluorescent substances. For example, one of the above-mentioned cDNA samples can be labeled with fluorescent substance Cy5, and the other with Cy3. The intensity of the each fluorescent signal shows the expression level of the gene encoding the polypeptide of the present invention in the subject and control respectively (Duggan et al., Nat. Genet. 21:10-14 (1999)).

On the other hand, when testing the translational level of a gene encoding a polypeptide of the present invention, polypeptide samples are first prepared from subjects. The amount of the polypeptide of the present invention comprised in the polypeptide sample is then measured, and compared with that of a control.

Exemplarily methods include SDS polyacrylamide electrophoresis methods; and methods utilizing antibodies binding to the polypeptides of the invention, like Western blotting, dot-blotting, immunoprecipitation, enzyme-linked immunosorbent assays (ELISA), and immunofluorescence.

When the expression level of a gene encoding a polypeptide of the present invention differs significantly from that of a control, the subject is judged to be infected with a disease related to gene expression abnormality, or to be in danger of disease onset.

### <Test drugs>

Furthermore, the present invention provides test drugs for diseases related to abnormal expression of a gene encoding a polypeptide of the present invention, or related to an abnormal activity of a polypeptide of the present invention.

An embodiment of a test drug of the present invention comprises an oligonucleotide comprising a chain length of at least 15 nucleotides, which hybridizes with a DNA comprising a polynucleotide encoding a polypeptide of the present invention, or its expression control region, as mentioned above. The oligonucleotides can be used in the above-mentioned test methods of the present invention as probes for detecting genes encoding polypeptides of the present invention, or their expression control region, or as primers for amplifying genes encoding polypeptides of the present invention, or their expression control region. The oligonucleotides of the present invention can be prepared, for example, by a commercially available oligonucleotide synthesizer. The probes can be also prepared as double-stranded DNA fragments, obtained by restriction enzyme treatments and the like. The oligonucleotides of the present invention are preferably appropriately labeled for use as a probe. Labeling methods include, for example, a labeling method that uses T4 polynucleotide kinase to phosphorylate the 5'-terminus of the oligonucleotide with ³²P; and a method of introducing substrate bases, which are labeled with isotopes such as ³²P, fluorescent dyes, biotin, and such, using random hexamer oligonucleotides and such as primers, and DNA polymerase such as Klenow enzyme (the random prime method, etc.).

Another embodiment of the test drugs of the present invention is a test drug comprising an antibody which binds to a polypeptide of the present invention, described below. In the above-mentioned test methods of the present invention, the antibodies are used to detect the polypeptides of the present invention. The form of the antibodies is not limited so long as they can detect a polypeptide of the present invention. Polyclonal antibodies and monoclonal antibodies are included as the antibodies for use in tests. The antibodies may be labeled as required.

For example, in addition to effective ingredients, oligonucleotides and antibodies, the above-mentioned test drugs may be mixed with sterilized water, physiological saline, vegetable oils, surfactants, lipids, solubilizers, buffers, protein stabilizers (such as BSA and gelatin), preservatives, and such, as necessary.

### <Antibodies>

The present invention provides antibodies that bind to a polypeptide of the present invention. Herein, the term "antibodies" refers to polyclonal antibodies, monoclonal antibodies, chimeric antibodies, single-stranded antibodies, humanized antibodies, and Fab fragments including Fab or other products of an immunoglobulin expression library.

A polypeptide of the present invention or its fragment, or analogs thereof, or a cell that expresses the same, can be used as an immunogen for producing antibodies that bind to a polypeptide of the present invention. The antibodies are preferably immunospecific to a polypeptide of the present invention. The term "immunospecific" means that an antibody has substantially higher affinity to polypeptides of the present invention compared to other polypeptides.

The antibodies binding to a polypeptide of the present invention can be prepared by conventional methods. For example, a polyclonal antibody can be obtained as follows: A polypeptide of the present invention, or a GST-fusion protein thereof, is administered to small animals, such as rabbits, to obtain serum. Polyclonal antibodies are prepared by purifying the serum by ammonium sulfate precipitation; a protein A or protein G column; DEAE ion exchange chromatography; an affinity column in which the polypeptide of the present invention is coupled; and such. On the other hand, monoclonal antibodies, for example, can be prepared as follows: A polypeptide of the present invention is administered to small animals such as mice, and their spleens are subsequently extirpated and ground down to separate the cells. The cells are then fused with mouse myeloma cells using reagents such as polyethylene glycol, and clones that produce antibodies binding to the polypeptide of the present invention are selected from these fused cells (hybridomas). The obtained hybridomas are then transplanted into mice peritoneal cavities, and ascites are collected from the mice. The monoclonal antibodies can be prepared by purifying the ascites using, for example, ammonium sulfate precipitation; a protein A or protein G column; DEAE ion exchange chromatography; an affinity column in which the polypeptides of the present invention are coupled; and such.

The antibodies of the present invention can be used to isolate, identify, and purify the polypeptides of the present invention, and the cells expressing them. Antibodies binding to a polypeptide of the present invention are thought to suppress the activities or expressions of the polypeptides of the present invention. Thus, compounds comprising effective amount of the antibodies are expected to be used as pharmaceutical compounds for treating patients in which the activities or expression of the polypeptides of the present invention are required to be suppressed. In addition, the antibodies can be also used to determine the expression level of a polypeptide of the present invention, to test for a disease related to abnormal expression of the polypeptide of the present invention.

The antibodies of the present invention can be used for isolating, identifying, and purifying the polypeptides of the present invention, and cells that express the same. Since an antibody that binds to the polypeptides of the present invention is considered to suppress the activity or expression of the polypeptide of the present invention, a composition comprising an effective amount of an antibody is expected to serve as a pharmaceutical composition for treating patients in need of suppression of an activity or expression of a polypeptide of the present invention. In addition, the antibodies can also be used for measuring the amount of expression of a polypeptide of the present invention, in tests for diseases related to abnormal expression of a polypeptide of the present invention.

### <Identification of candidate compounds for therapeutic agents>

The polypeptides of the present invention can be used in screening for candidate therapeutic agent compounds for diseases related to abnormal expression of a polypeptide of the present invention. These molecules for identification may be naturally occurring or artificially synthesized structural or functional mimetics. The polypeptides of the present invention are involved in numerous biological functions, including pathologies. Thus, it is preferable to discover compounds that activate the polypeptides of the present invention, as well as compounds that can inhibit activation of the polypeptides of the present invention.

The present invention provides methods of screening for candidate compounds of therapeutic agents for diseases related to abnormal expression of genes encoding polypeptides of the present invention, or related to abnormal activity of the polypeptides of the present invention. These methods comprise the steps of: first contacting a polypeptide of the present invention with a candidate compound; measuring the galactose transferring activity of the polypeptide of the present invention; and selecting compounds that change (increase or suppress) galactose transferring activity compared to when the test compound is not contacted.

Using the aforementioned screening methods of the present invention, compounds isolated as those that increase galactose transferring activity of the polypeptides of the present invention are useful as therapeutic agents for diseases such as IgA nephropathy and Tn syndrome. On the other hand, compounds isolated as compounds that suppress the galactose transferring activity of the polypeptides of the present invention are useful as enzyme activity inhibitors, such as anti-inflammatory agents and anticancer agents. Moreover, compounds designed as drugs by crystallizing the polypeptides of the present invention can also be used as enzyme activity inhibitors. In addition, these compounds can also be used as test compounds in the aforementioned screening methods of the present invention.

Galactose transferring activity can be measured using, for example, methods previously described. The test compounds are not particularly limited, and various known compounds and peptides (e.g., those registered in the Chemical File) or random peptide groups prepared by applying the phage display method (J. Mol. Biol. (1991) 222, 301-310), and such can be used. In addition, the culture supernatants of microorganisms and naturally occurring components derived from plants and marine organisms can also be screened. Other examples comprise, but are not limited to, body tissue extracts from the brain and such, cell extract liquids, and gene library expression products.

### <Pharmaceutical compositions for treating diseases>

The present invention provides pharmaceutical compositions for treating patients who require increased or suppressed activity or expression of a polypeptide of the present invention.

A polynucleotide of the present invention, a vector wherein a polynucleotide of the present invention is inserted, and a polypeptide of the present invention can be used as effective ingredients for the pharmaceutical compositions for increasing the activity or expression of the polypeptides of the present invention. On the other hand, an antibody against a polypeptide of the present invention or a polynucleotide suppressing the expression of a gene encoding an endogenous polypeptide of the present invention *in vivo,* can be used as an effective ingredient of a pharmaceutical composition for suppressing the activity or expression of a polypeptide of the present invention. The above-described antisense polynucleotides and ribozymes can be used as the polynucleotides.

When the therapeutic compounds are used as pharmaceutical agents, they can be administered as pharmaceutical compositions prepared by known pharmaceutical methods, and can also themselves be directly administered to a patient. For example, they can be used in pharmaceutical compositions obtained by mixing an active ingredient with a pharmacologically acceptable carrier (such as an excipient, binder, disintegrator, flavor, corrigent, emulsifier, diluent, or solubilizer), or can be formulated into forms suitable for oral or parenteral administration, such as a tablet, pill, powder, granule, capsule, troche, syrup, liquid, emulsion, suspension, injection (such as liquid, and suspension), suppository, inhalant, percutaneous absorbent, eye drop, eye ointment or the like.

Administration to a patient can typically be carried out by methods known to those skilled in the art, such as intraarterial injection, intravenous injection, subcutaneous injection, and the like. Although the dosage varies depending on the weight and age of the patient, as well as administration methods and the like, those skilled in the art can suitably select appropriate doses. Further, if the compound can be encoded by a DNA, gene therapy can be also carried out by introducing the DNA to a gene therapy vector.

Gene therapy vectors include, for example, viral vectors such as retroviral vectors, adenoviral vectors, adeno-associated viral vectors; and non-viral vectors such as liposomes; and such. A target DNA can be administered to a patient by *ex vivo* methods and *in vivo* methods, utilizing such vectors.

### <Genetically altered animals>

The present invention provides genetically altered animals in which the expression of the C1Gal-T2 protein of the present invention has been artificially altered.

The aforementioned C1Gal-T2 protein is not necessarily limited to a polypeptide comprising the amino acid sequence of SEQ ID NO: 2, but rather comprises a polypeptide functionally equivalent to a polypeptide endogenously comprised by an animal to be genetically altered, as well as that animal's homologue protein.

In the present invention, "expression of the ClGal-T2 gene of the present invention has been artificially altered" normally refers to a state in which the degree of expression of a gene that encodes C1Gal-T2 protein is altered by a mutation such as the addition, insertion, deletion, or substitution of a nucleotide; a state in which the number of C1Gal-T2 gene copies is altered; or a state in which an exogenous DNA that encodes C1Gal-T2 is introduced. For example, non-human animals in which C1Gal-T2 gene is knocked-out, and non-human C1Gal-T2 transgenic animals, are also included in the genetically altered non-human animals of the present invention. The genetically mutated sites are not particularly limited, as long as they facilitate the expression of the gene to be altered, and these sites include exon sites and promoter sites, for example.

In addition, the expression of mutant C1Gal-T2 proteins, in which the function of normal C1Gal-T2 protein is increased or decreased, is also included as "altered" expression of the ClGal-T2 gene. Cases are known where the expression of a gene increases if a mutation is in the gene's expression control region, such as a promoter site. Thus, in one embodiment of an "alteration" of the present invention, the increased C1Gal-T2 gene expression is due to a mutation in its expression control region.

In addition, the present invention suitably provides, for example, non-human animals in which an exogenous expressible DNA that encodes C1Gal-T2 protein is introduced, as well as a non-human animal in which expression of endogenous C1Gal-T2 gene is increased. Namely, in a preferable embodiment of the present invention, genetically altered non-human animals are provided in which an exogenous DNA encoding C1Gal-T2 protein has been introduced.

The genetically altered non-human animals of the present invention can be used in methods of screening for therapeutic agents for diseases such as IgA nephropathy and Tn syndrome, and are extremely useful for such purposes.

In the methods of the present invention the term "non-human animal" refers to vertebrates except humans, and invertebrates. Non-human animals suited to the artificial alteration of gene expression using gene engineering technology comprise non-human mammals and insects, preferably non-human mammals (e.g., rodents such as mice and rats), and most preferably mice.

Methods for producing genetically altered animals are well known. For example, genetically altered animals can be obtained by the methods described in Proc. Natl. Acad. Sci. USA 77: 7380-7384 (1980).

For example, methods for producing genetically altered non-human animals (transgenic animals), which have been introduced with an exogenous DNA encoding a C1Gal-T2 protein, comprise the following steps: a DNA encoding C1Gal-T2 is first introduced into an animal's totipotent cells; these cells are developed into individuals; and individuals in which the introduced gene has been incorporated into somatic cells and reproductive cells are selected from the resulting individuals. Examples of totipotent cells into which genes are introduced include cultured cells such as ES cells comprising multi-potency, as well as fertilized eggs and early embryo cells. By adapting known methods, one of ordinary skill in the art can produce genetically altered animals in which the expression of a desired gene is altered.

The aforementioned "DNA encoding C1Gal-T2" is typically a recombinant gene construct linked to a promoter that is capable of expression in the animal cells into which the DNA is introduced (an expression vector). Recombinant gene constructs of the present invention can be constructed by using a suitable host, inserting an aforementioned DNA encoding C1Gal-T2 into a clonable vector, inserting a promoter upstream of that DNA, and then cloning.

There are no particular limitations on the promoter to be used in the present invention, as long as the promoter can be expressed in animal cells. For example, the promoters include promoters derived from mammalian cells, as well as viral promoters of cytomegaloviruses, retroviruses, polyoma viruses, adenoviruses, and Simian virus 40 (SV40). For example, when using an SV40 promoter, the aforementioned constructs can be constructed by the methods of Mulligan, et al. (Nature (1990) 277, 108).

A preferable example of a vector that can be used in the present invention is a CAG vector (e.g., pCAGGS). In addition to this vector, expression vectors commonly known to those skilled in the art can also be used, as long as they can induce wide expression of an introduced gene *in vivo.* Specifically, preferable examples of useable vectors other than pCAGGS are vectors comprising a promoter of human polypeptide chain elongation factor 1 alpha (hEF1α) (Hanaoka, K. et al.: Differentiation 1991: 48: 183-189), or CMV promoter-enhancer (Schmidt, E.V. et al.: Mol. Cell. Biol. 1990: 10: 4406-4411).

In addition, CMV-derived enhancers are known to enhance the expression of exogenous genes in mammals. Thus, the enhancers can be inserted into the aforementioned constructs of the present invention in order to enhance expression of an exogenous gene.

When constructing a recombinant gene construct comprising these genes, a vector that comprises an enhancer and a promoter, downstream of which is a multi-cloning site for inserting an exogenous gene, can be used. A vector comprising this type of structure can be constructed based on pCAGGS, for example.

The recombinant gene constructs, cleaved from the aforementioned vectors by suitable restriction enzymes, are used for the production of adequately purified genetically altered animals. Normally, genetically altered animals are produced by introducing the aforementioned constructs into unfertilized eggs, fertilized eggs, sperm, or embryonic cells comprising primitive cells of the same. Normally, cells to which the constructs are introduced are those in the embryonic generation stage of non-human mammal generation, and more specifically, those cells in the single cell stage or fertilized egg stage are usually at the 8-cell stage or earlier. Methods for introducing an aforementioned construct, such as calcium phosphate methods, electric pulse methods, lipofection methods, aggregation methods, microinjection methods, particle gun methods and DEAE-dextran methods, are well-known. Moreover, genetically altered animals can be produced by fusing the transformed cells obtained in this manner with the aforementioned embryo cells.

Cells that are introduced with the aforementioned construct can be derived from any non-human animal that allows production of a genetically altered animal. Specific examples of such cells are those of mice, rats, hamsters, guinea pigs, rabbits, goats, sheep, pigs, cows, dogs, and cats. In the case of mice, fertilized eggs to which the constructs can be introduced can be recovered by, for example, mating a normal male mouse with a female mouse to which a fertility drug is administered. Constructs are typically introduced into mouse fertilized eggs by microinjection into the male pronucleus. After being cultured outside the body, cells into which the construct is successfully introduced are transplanted to the uterine tube of a surrogate mother, resulting in the birth of a genetically altered chimeric animal. A female, put in a pseudopregnant state by mating with a male whose seminal ducts have been severed, is ordinarily used as a surrogate mother.

After confirming that a DNA encoding C1Gal-T2 is introduced in a resulting genetically altered chimeric animal, the animal is mated with a normal animal to produce F1 animals. A number of copies of the exogenous DNA that was introduced as a construct are typically incorporated in series in the same portion of the genome. Normally, a greater number of incorporated copies causes greater gene expression, and thus a significant phenotype can be expected. Whether or not a DNA encoding C1Gal-T2 is incorporated in the proper direction in the somatic cell genome can be confirmed by PCR, using a primer specific to the construct or by Southern blotting using a specific probe.

Of the F1 animals born as a result of this mating, those heterozygotes that comprise an exogenous gene (a DNA encoding C1Gal-T2) in their somatic cells are genetically altered animals capable of transmitting the exogenous gene (a DNA encoding C1Gal-T2) to reproductive cells. F2 homozygote animals can be obtained by selecting, as parents, F1 animals retaining the exogenous gene (a DNA encoding C1Gal-T2) in their somatic cells.

The animals with an altered C1Gal-T2 gene of the present invention may be of any generation of the aforementioned genetically altered animals, as long as the expression of C1Gal-T2 is altered. For example, a genetically altered animal heterogeneously retaining an exogenous C1Gal-T2 DNA can be used.

In addition, examples of means for artificially suppressing expression of the C1Gal-T2 gene include methods for deleting all or a portion of the C1Gal-T2 gene, and methods for deleting all or a portion of an expression control region of C1Gal-T2 gene. Preferably, a method for inactivating a C1Gal-T2 gene by inserting an exogenous gene into one or both of the C1Gal-T2 gene pairs is used.

Animals of the present invention in which expression of a C1Gal-T2 gene is artificially suppressed, such as C1Gal-T2 gene knockout animals, can be produced by common genetic engineering technology known in the art. Mice are exemplary non-human animals, and the knockout mice of the present invention can be produced as described below. First, DNAs comprising an exon portion of a C1Gal-T2 gene are isolated from the mice. Suitable marker genes are inserted into the DNA fragments to construct targeting vectors. The targeting vectors are introduced into mouse ES cell lines by electroporation methods or such, and those cells lines in which homologous recombination has occurred are selected. Antibiotic-resistant genes, such as neomycin-resistant genes, are preferably used as the inserted marker gene. When an antibiotic-resistant gene has been inserted, cell lines in which homologous recombinations have occurred can be selected by simply culture on a medium comprising the corresponding antibiotic. In addition, a thymidine kinase gene or such can be linked to the targeting vector to carry out selection more efficiently. As a result, those cells lines in which non-homologous recombination has occurred can be omitted. In addition, cell lines in which one of C1Gal-T2 gene pairs has been inactivated can be efficiently obtained by testing for homologous recombinants using PCR and Southern blotting.

Due to the risk of destroying unknown genes by gene insertion at locations other than at a site of homologous recombination, it is preferable to provide chimeric cells using a plurality of clones when selecting cell lines in which homologous recombination has occurred. Chimeric mice can be obtained by injecting the resulting ES cell lines into mouse blastoderms. By mating these chimeric mice, mice in which one of C1Gal-T2 gene pairs is inactivated can be obtained. Furthermore, these obtained mice can be mated to obtain mice in which both C1Gal-T2 gene pairs are inactivated. In addition to mice, other gene knockout animals for which ES cells have been established can also be obtained using similar techniques.

In addition, ES cell lines in which both ClGal-T2 gene pairs have been inactivated can be acquired by methods as described below. Namely, by culturing an ES cell line in which one C1Gal-T2 gene pair has been inactivated in a medium comprising a high concentration of antibiotics, a cell line can be obtained in which the other gene pair is also inactivated, i.e., in which both C1Gal-T2 gene pairs are inactivated. In addition, these ES cell line can also be produced by selecting an ES cell line in which one gene pair has been inactivated, reintroducing a targeting vector to the cell line, and then selecting those cell lines in which homologous recombination has occurred. The marker genes to be inserted into the targeting vector are preferably different from the previously described marker gene.

Moreover, the animals of the present invention in which expression of C1Gal-T2 gene is artificially inhibited can also be produced by introducing a following polynucleotide into an animal:
(a) an antisense polynucleotide to a transcription product of a gene encoding C1Gal-T2 protein, or a portion thereof;
(b) a polynucleotide comprising ribozyme activity that specifically cleaves a transcription product of a gene encoding C1Gal-T2 protein; and
(c) a polynucleotide that inhibits expression of a gene encoding C1Gal-T2 protein by RNAi effects.

The present invention also provides cells established from the genetically altered animals of the present invention. Known methods can be used to establish cell lines derived from the genetically altered animals of the present invention. For example, a cell line can be established in rodents by primary culturing method of fetal cells (New Biochemistry Experimental Course (Shin Seikagaku Jikken Kouza), 18, 125-129, Tokyo Kagaku Dojin; and Manual of Mouse Embryo Manipulation, 262-264, Kindai Publishing).

For example, genetically altered animals of the present invention, and cell lines established from these animals, can be used to analyze specific functions of the C1Gal-T2 gene, such as the action mechanism of compounds that regulate C1Gal-T2 gene expression. The use of cells established from the tissues of genetically altered animals enables more detailed study of the action of test compounds in various tissues.

Moreover, the present invention provides methods of screening for compounds that change the activity or expression of C1Gal-T2 proteins of the present invention.

A preferable embodiment of the present invention is a screening method that uses, as an indicator, a change in the activity or expression of C1Gal-T2 proteins in the genetically altered non-human animals of the present invention, or in the cells of the present invention. Such methods comprise a first step of administering test compounds to the genetically altered non-human animals of the present invention, or contacting test compounds with cells of the present invention (Step (a)).

Examples of test compounds used in the present methods include single compounds such as naturally-occurring compounds, organic compounds, inorganic compounds, proteins, and peptides, as well as compound libraries, expression products of gene libraries, cell extracts, cell culture supernatants, microbial fermentation products, marine organism extracts, and plant extracts.

In the aforementioned step (a), test compounds can be orally administered or injected to the genetically altered animals of the present invention, without limitation. When the test compound is a protein, viral vectors comprising a gene encoding the protein can be constructed, and the gene can be introduced into a genetically altered animal of the present invention by utilizing this infectious ability.

Although the step of "contacting" in the aforementioned method is ordinarily carried out by adding test compounds to a cell culture of the present invention, it is not particularly limited thereto. When the test compound is a protein, the step of "contacting" can be carried out by, for example, introducing DNA vectors that express the protein into the cells.

In the aforementioned methods, the activity or expression amount of ClGal-T2 protein in the aforementioned genetically altered animals or cells of the present invention is then measured (Step (b)).

The aforementioned term "activity" normally refers to galactose transferring activity. This activity can be measured by an above-described method.

In addition, in the present invention, protein expression can be measured by methods known in the art. For example, the level of C1Gal-T2 gene transcription can be measured by using common methods to extract mRNAs from the cells of the present invention that express C1Gal-T2 gene, or cells of the genetically altered animals of the present invention; and then carrying out Northern hybridization or RT-PCR using the mRNAs as templates. In addition, the level of C1Gal-T2 gene translation can be measured by recovering protein fractions from cells that express C1Gal-T2 gene; and detecting expression of C1Gal-T2 protein by an electrophoresis method such as SDS-PAGE. Moreover, the translation amount of C1Gal-T2 gene can be measured by Western blotting using an antibody to C1Gal-T2 protein to detect protein expression. The antibodies used to detect C1Gal-T2 protein are not particularly limited, as long as they are detectable, and they may be both monoclonal and polyclonal antibodies, for example.

In the present invention, compounds that change the activity or expression amount of C1Gal-T2 proteins of the present invention are next selected by comparison with a case where the test compounds were not administered (Step (c)). Compounds selected in this manner are expected to serve as pharmaceutical candidate compounds for treating or preventing diseases caused by a change in an activity of a protein of the present invention. Although the aforementioned term "diseases" comprises IgA nephropathy and Tn syndrome, it is not particularly limited provided they are caused by a change in the expression amount or activity of the proteins of the present invention. Compounds acquired by the screening methods of the present invention are also included in the present invention.

### Brief Description of the Drawings

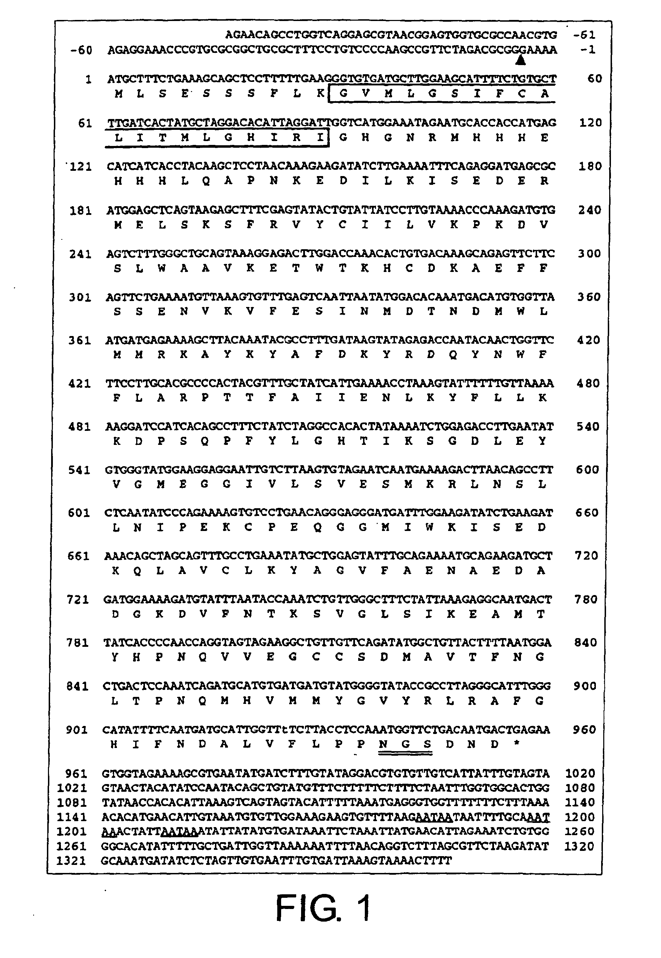

Fig. 1 shows the nucleotide sequence of C1Gal-T2 cDNA and the amino acid sequence encoded by this nucleotide sequence. The predicted transmembrane domain is shown in a box, and the polyadenylation signals are underlined with single lines. The glycosylation site linked to asparagine is underlined with double lines. The arrow indicates a splicing site.

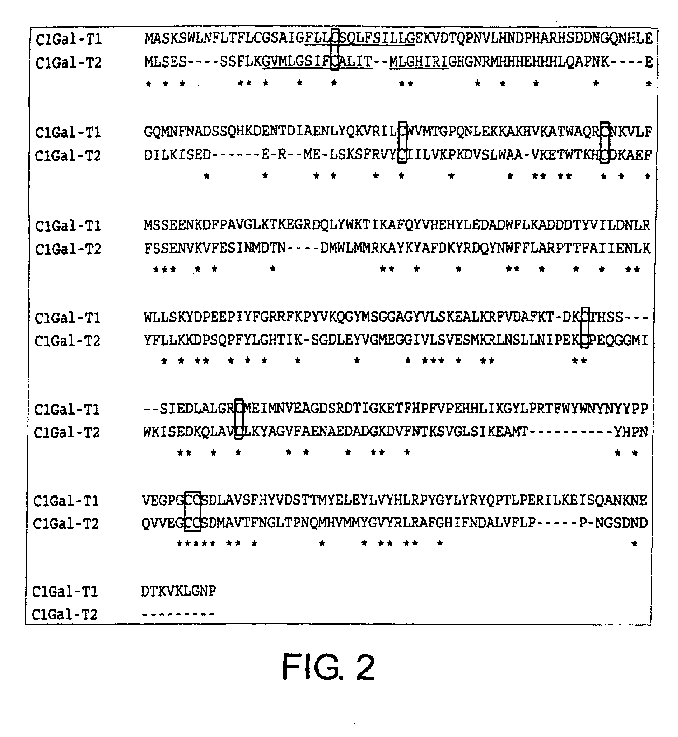

Fig. 2 compares the amino acid sequences of ClGal-T2 and C1Gal-T1. The preserved cysteine residues are shown in boxes.

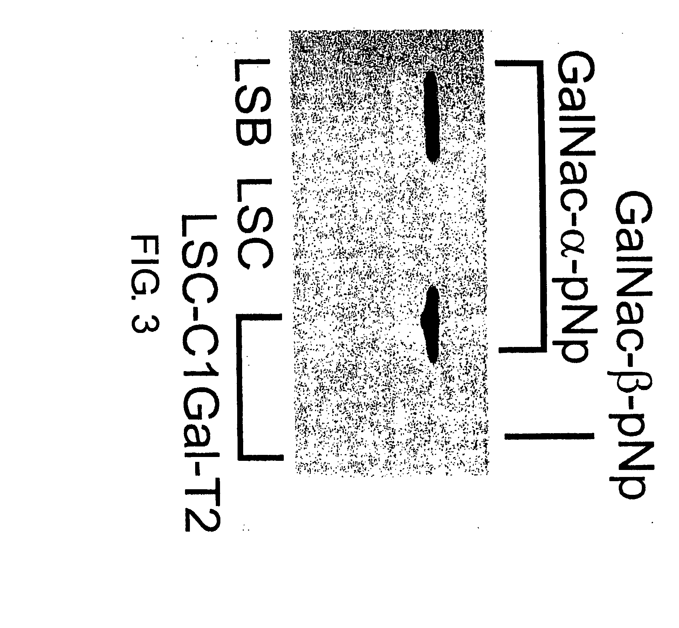

Fig. 3 is a photograph showing the results of measuring the TLC plate with an FLA3000 Image Analyzer.

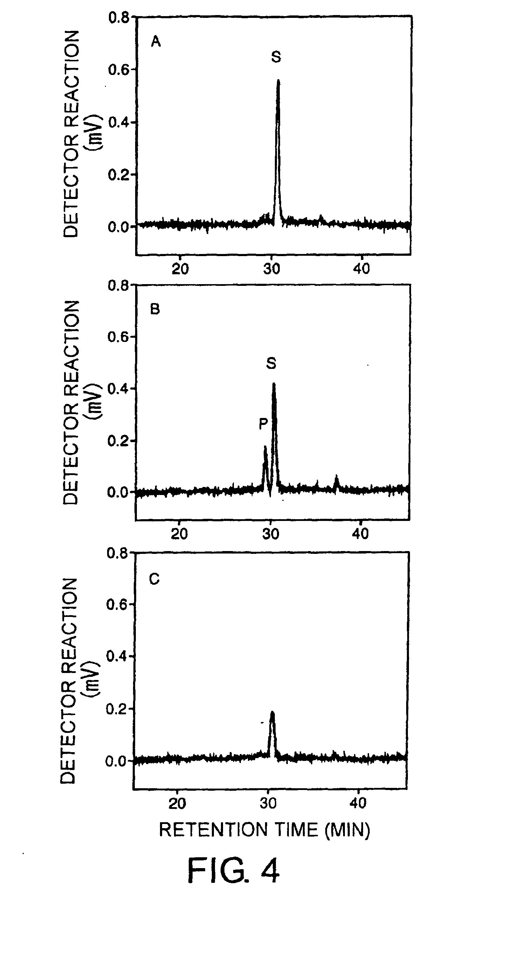

Fig. 4 shows the results of HPLC analysis of reaction products derived from 11-GalNAc-HP. Peak S indicates the location of eluted Cy5-labeled 11-GalNAc-HP (Panel A). Panel B shows the reaction product (Peak P) of LSC-C1Gal-T2 cell lysate and the microsome fraction of the remaining substrate (Peak S). Panel C shows the reaction product (Peak P) following digestion by β1,3-galactosidase.

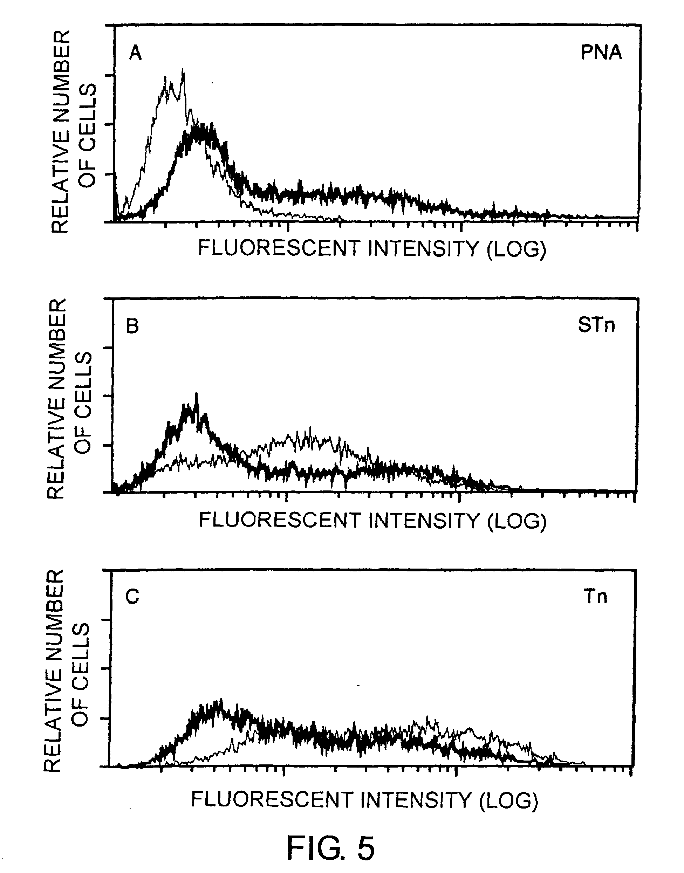

Fig. 5 shows the results of flow cytometry analysis of LSC cells transfected with human C1Gal-T2. The expression of T-related antigen on the surface of the LSC-hC1Gal-T2 transfectant was analyzed by flow cytometry. The narrow line in each panel indicates the results of mock transfection using LSC cells stained with various lectins or antibodies. Each panel shows stable LSC cell transfectants stained with PNA lectin (A), HBSTn1 (sialyl Tn) (B), and HB-T1 (Tn) (C).

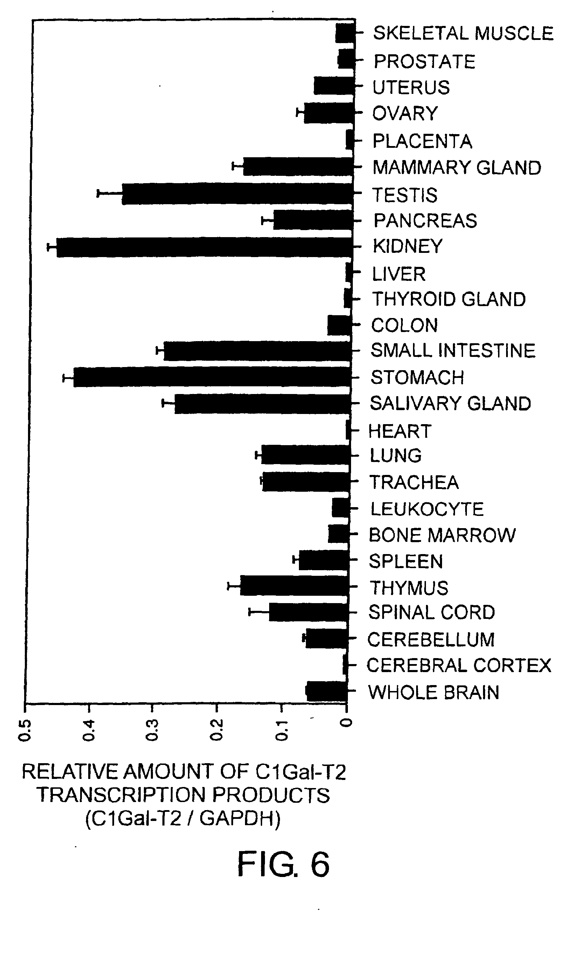

Fig. 6 shows the results of quantitative analysis of human C1Gal-T2 transcription products in various human cells by real-time PCR. The standard curves for C1Gal-T2 and GAPDH were generated from a dilution series of each plasmid. The expression level of C1Gal-T2 transcription product was standardized according to the expression level of the GAPDH transcription product.

Fig. 7 shows eight types of glycosyltransferase core structure.

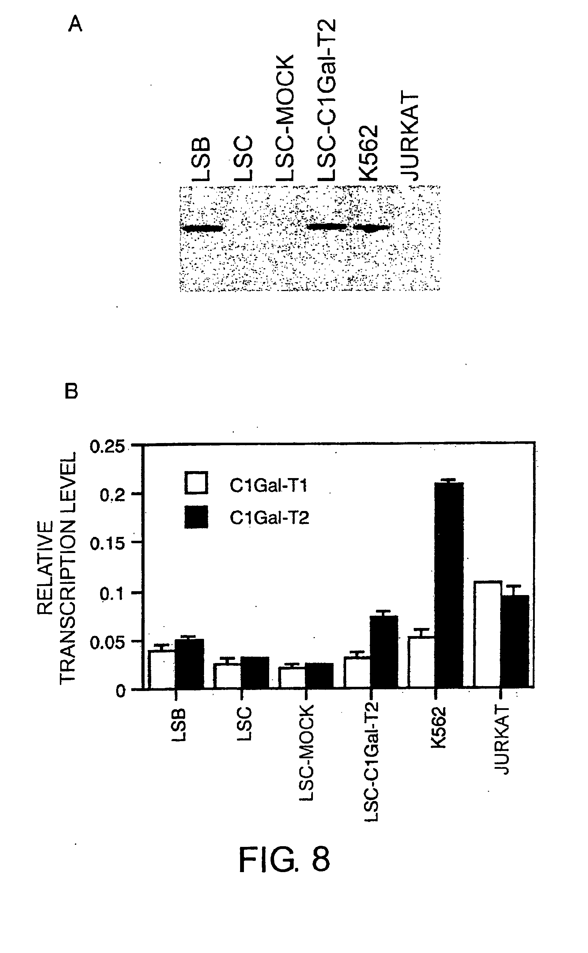

Fig. 8 shows a photograph (A) of core 1 synthesis activity on GalNAc-α-pNp in various cell lines and LSC-C1Gal-T2, and a graph (B) of the expression levels of transcripts of C1Gal-T1 and C1Gal-T2.

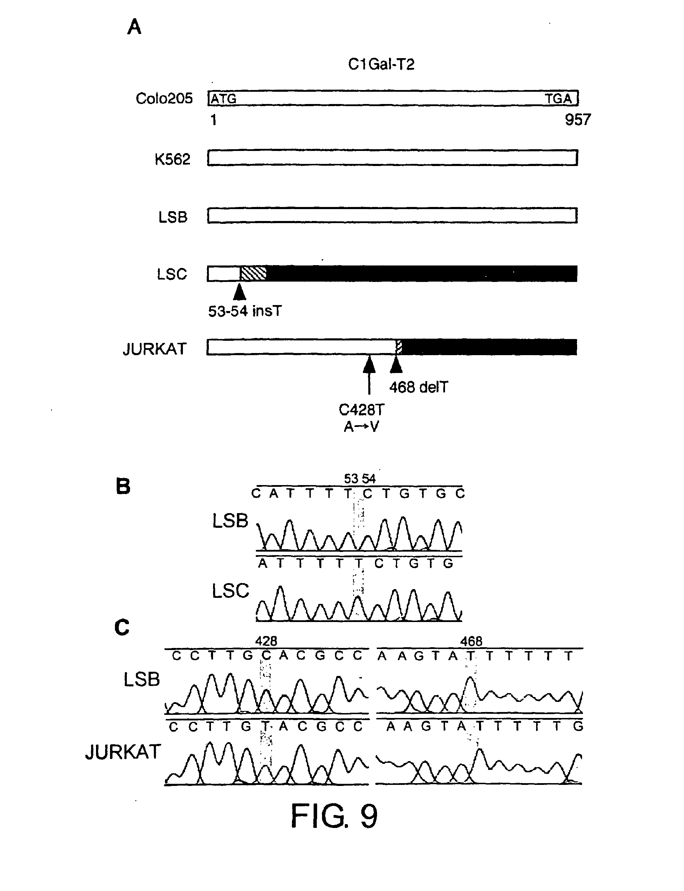

Fig. 9A shows a schematic representation of a comparison of the C1GalT1-2, K562, LSB, LSC, and Jurkat sequences. Fig. 9B shows a comparison of LSB and LSC. Fig. 9C shows a comparison of LSB and Jurkat.

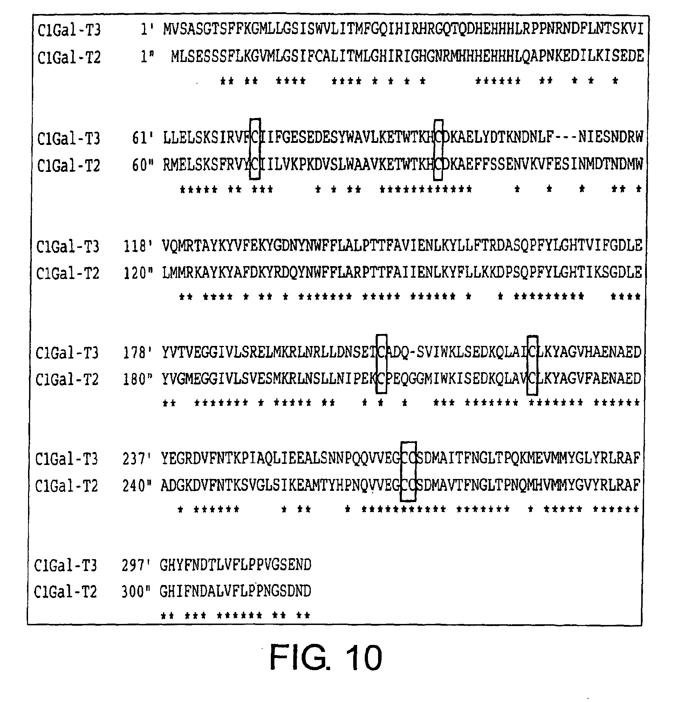

Fig. 10 shows a comparison of the amino acid sequences of C1Gal-T3 and C1Gal-T2.

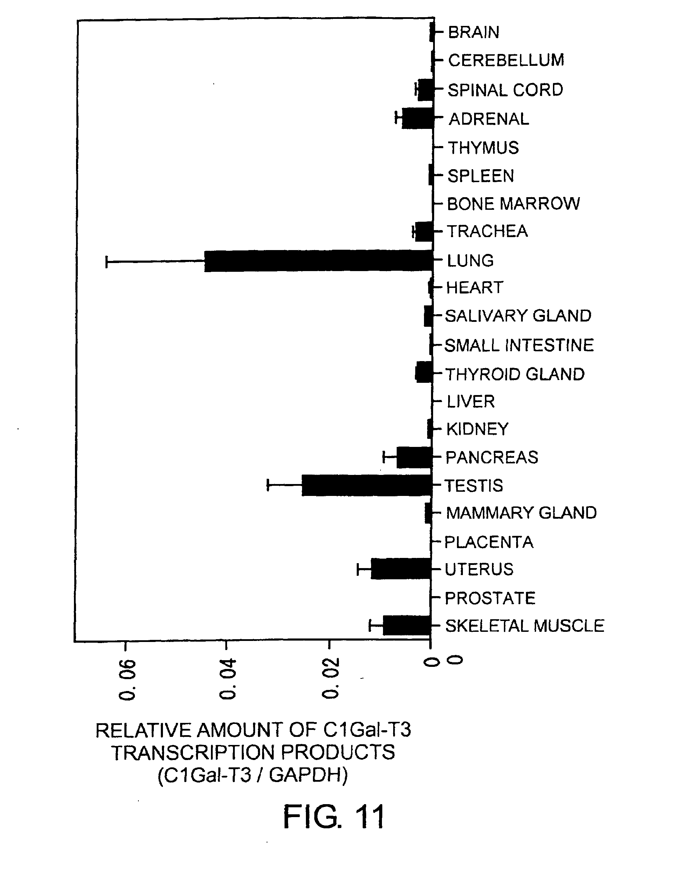

Fig. 11 shows the results of quantitative analysis of expression of human C1Gal-T3 transcription product in various human tissues by quantitative PCR. GAPDH gene was used as the standard for quantification. The expression amount of each gene was standardized by using a template DNA at a known concentration to generate a quantification standard curve.

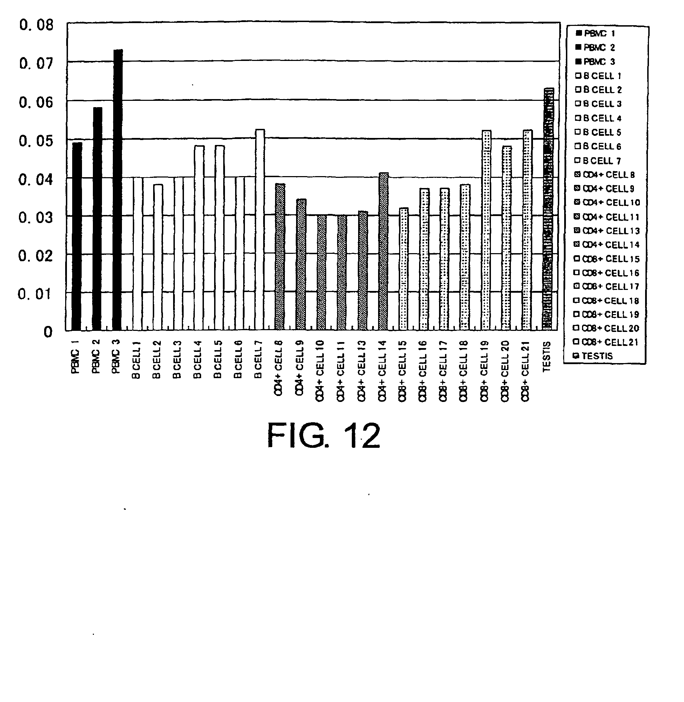

Fig. 12 shows the expression of C1Gal-T3 in blood cells, showing the results of quantitative analysis of human C1Gal-T3 transcription product in fractions of human peripheral blood mononuclear cells, B cells, helper T cells, and killer T cells, as determined by real-time PCR. Peripheral blood mononuclear cells were isolated from the whole blood of healthy volunteers using Ficol. The B cell, helper T cell, and killer T cell fractions were obtained from peripheral blood mononuclear cells using beads coupled with anti-CD19, anti-CD4, and anti-CD8 antibodies, respectively. Standard curves for C1Gal-T3 and GAPDH were generated from dilution series for each plasmid. The expression levels of C1Gal-T3 transcription products were standardized according to the expression level of the GAPDH transcription product. As a control, testis, which express a comparatively high level of C1Gal-T3, were also subjected to analysis.

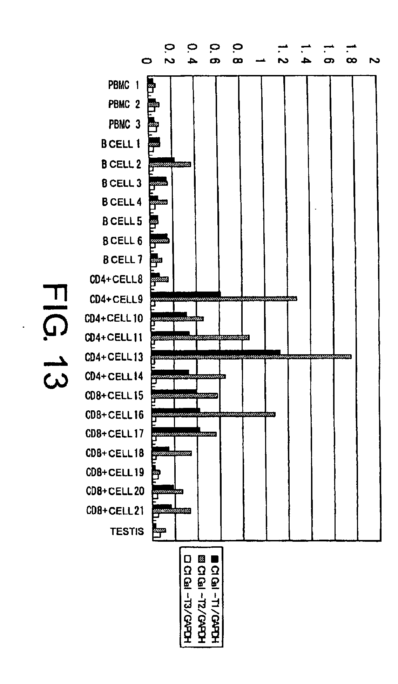

Fig. 13 shows the expression of C1Gal-T1, -T2, and -T3 in blood cells. The transcription products of C1Gal-T1 and C1Gal-T2 were quantitatively analyzed in the same manner as the aforementioned C1Gal-T3, and the three transcripts were compared.

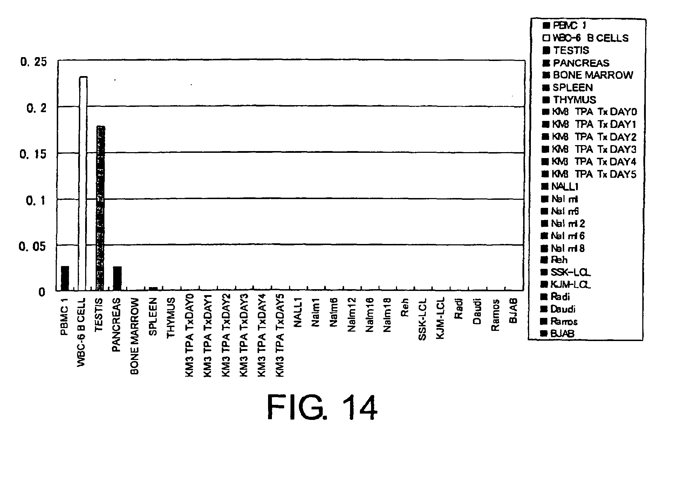

Fig. 14 shows the expression of C1Gal-T3 in various cell lines derived from human B cells. Since expression of C1Gal-T3 was observed in B cells (Figs. 12 and 13), the transcription products of human C1Gal-T3 in each cell line were quantitatively analyzed by real-time PCR.

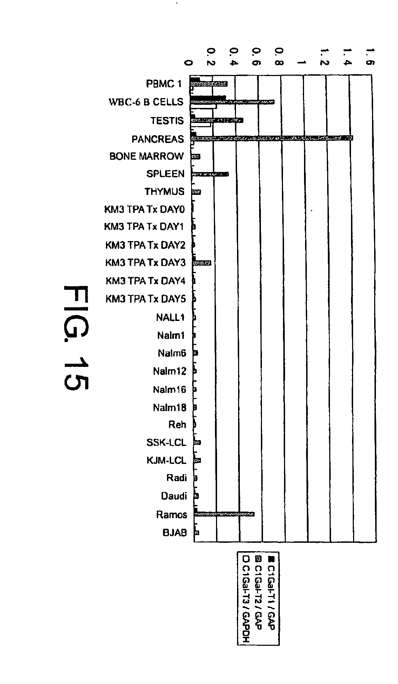

Fig. 15 shows the expression of C1Gal-T1, -T2, and -T3 in various cells lines derived from human B cells. The transcription products of C1Gal-T1 and C1Gal-T2 were quantitatively analyzed in the same manner as the aforementioned C1Gal-T3, and the three transcripts were compared.

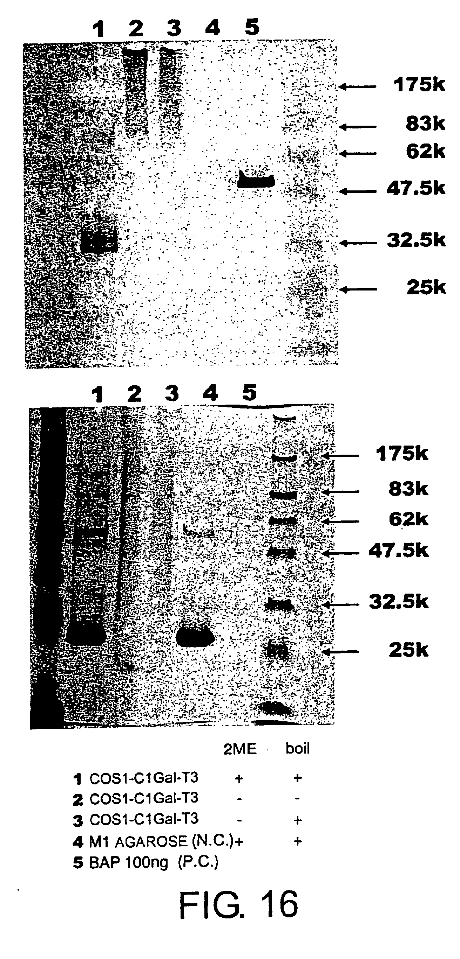

Fig. 16 shows two photographs of the results of transfection of C1Gal-T3 into COS-1 cells. These photographs show the results of SDS-PAGE of C1Gal-T3, which was purified from the culture supernatant of COS-1 cells transfected with pFLAG-CMV3-C1Gal-T3 using M1 agarose to which anti-FLAG antibodies were bound. A 12.5% acrylamide gel and HRP-anti-FLAG antibodies diluted 1000 times were used. The explanations of each lane 1 to 5 are shown below the photographs. The upper photograph shows the results of Western blotting with anti-FLAG antibody. The lower photograph shows the results of Coumassie staining.

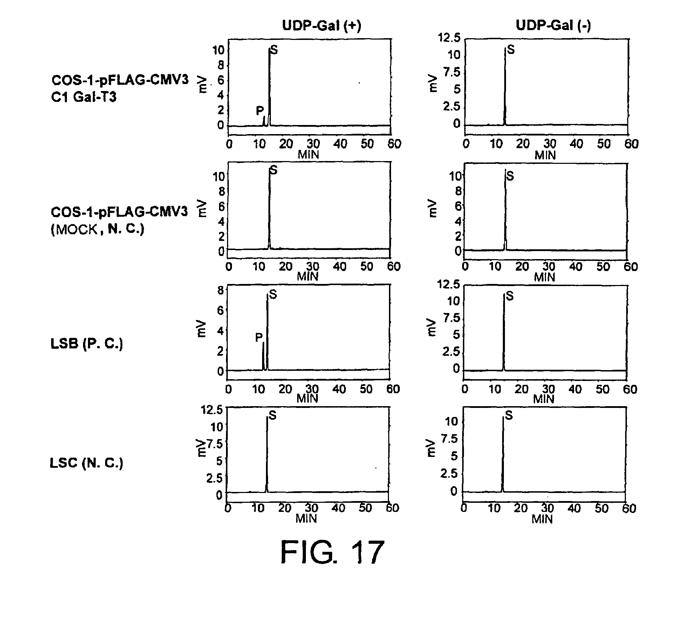

Fig. 17 shows the results of HPLC analysis for core 1 synthesis activity of COS-1-C1Gal-T3 transfectants on GalNAc-peptide. Starting from the top and moving down, the results are those when using the following enzyme sources: C1Gal-T3 purified using FLAG tag from the culture supernatant of COS-1-pFLAG-CMV3-ClGal-T3 transfectants, a fraction purified with Flag tag from the culture supernatant of COS-1-pFLAG-CMV3 transfectant (the mock vector-only transfectant), cell extract of cell line LSB comprising core 1 synthesis activity (positive control), and cell extract of cell line LSC not comprising core 1 synthesis activity (negative control). Fluorescent-labeled GalNAc-Ser was used as the receptor. The graphs on the right side represent controls whereby the enzyme reactions were carried out without adding donor substrates. In the graphs peak S represents the acceptor substrate peak, while peak P represents the product peak detected as a result of the enzyme reaction.

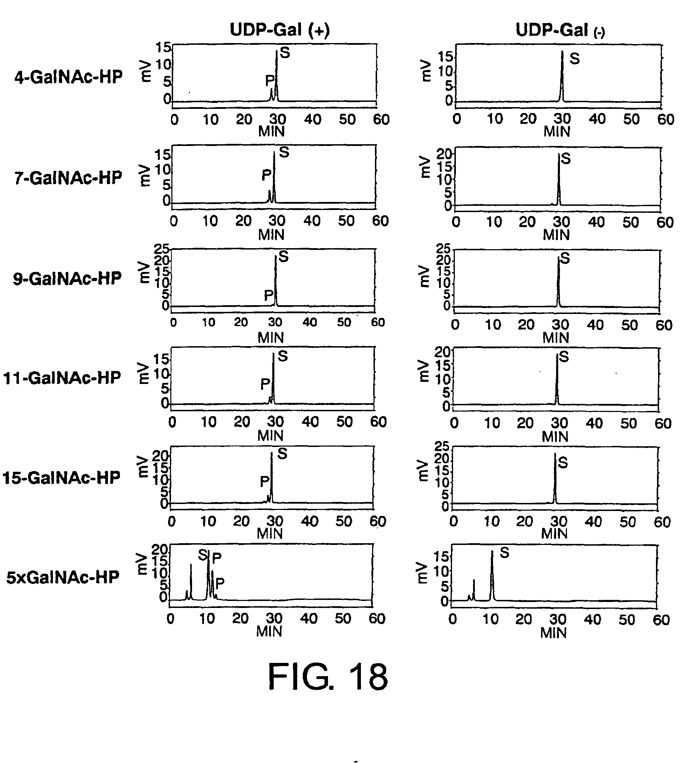

Fig. 18 shows the results of HPLC analyses, the enzyme source for all of which was ClGal-T3 purified with FLAG tag from the culture supernatant of COS-1-pFLAG-CMV3-C1Gal-T3 transfectant. The acceptor substrates are shown to the left of the chromatograms. In the graphs, HP indicates a synthetic peptide that mimics the amino acid sequence of the IgA1 hinge site, and the numbers such as 4 and 7 indicate the locations of amino acids to which GalNAc is added. For example, 4-GalNAc-HP represents an IgA1 hinge peptide in which GalNAc is added to the fourth amino acid from the N terminus. However, 5xGalNAc-HP represents a peptide in which GalNAcs are bound to all five locations: 4, 7, 9, 11, and 15.

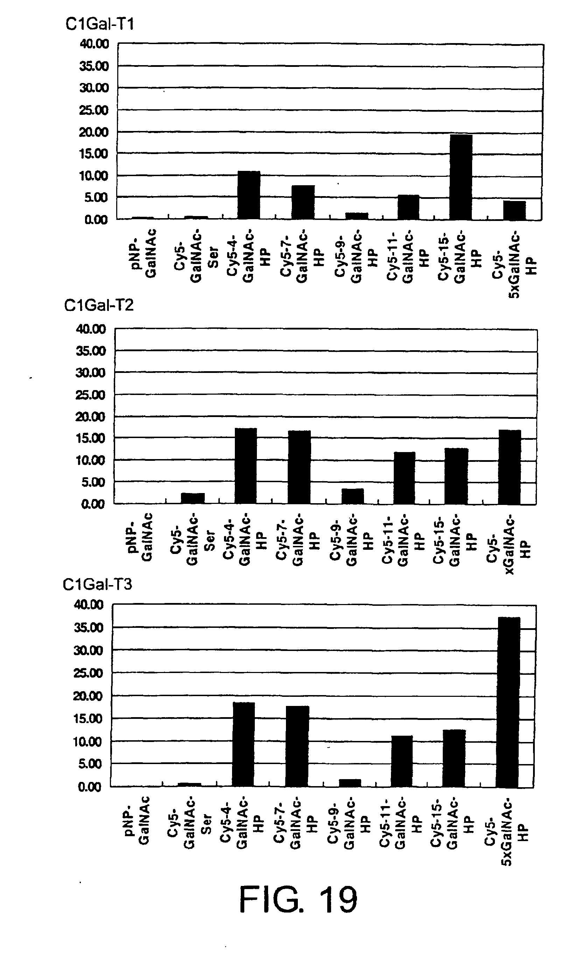

Fig. 19 shows comparisons of C1Gal-T1, -T2, and -T3 substrate specificities. The enzyme sources used for the analysis of the core 1 synthesis activity on each acceptor substrate were microsome fractions of LSC-C1Gal-T1 and LSC-C1Gal-T2, and purified enzyme derived from COS-1-C1Gal-T3.

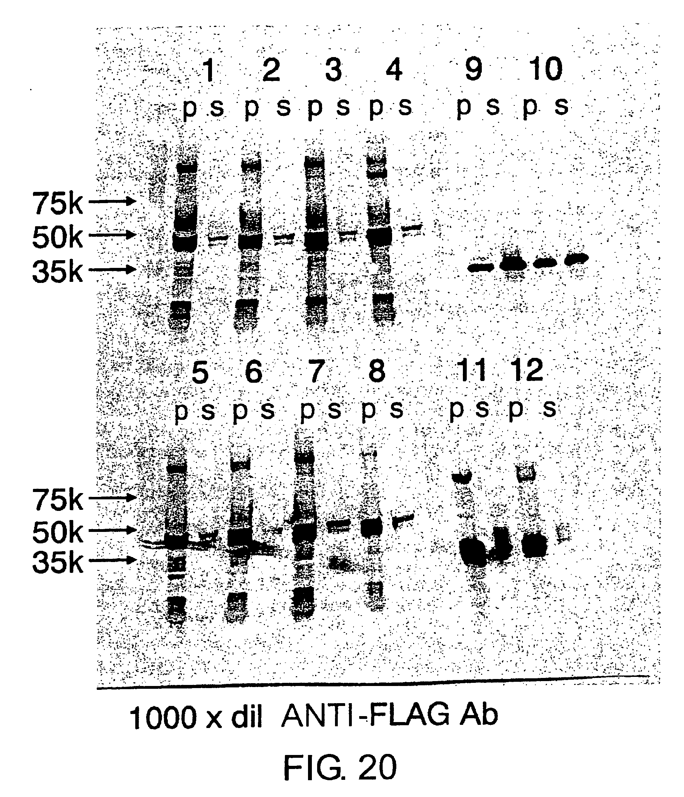

Fig. 20 is a photograph showing the co-expression of ClGal-T1, -T2, and -T3. C1Gal-T1, -T2, and -T3 were co-expressed in 293T cells in the combinations shown in the photograph. Western blotting was then carried out using anti-FLAG antibodies of C1Gal-T1, -T2, and -T3 purified from the resulting culture supernatant and cells. x1000-diluted anti-FLAG antibodies were used.

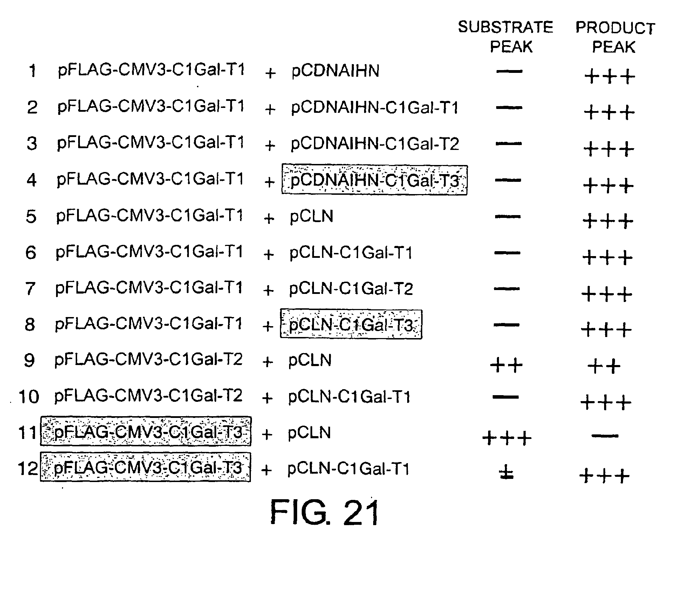

Fig. 21 shows the results of analyzing core 1 synthesis activity using co-expressed 293T transfectants of C1Gal-T1, -T2, and -T3. Core 1 synthesis activity on GalNAc-peptide was analyzed by HPLC using C1Gal-T purified from the culture supernatant of each 293T transfectant. Acceptor substrate, IgA hinge peptide attached to one GalNAc; N.C., No donor substrate.

### Best Mode for Carrying Out the Invention

The present invention is specifically illustrated below with reference to Examples, but it is not to be construed as being limited thereto.

### [Example 1] Identification of novel galactosyltransferase C1Gal-T2

Public databases were searched using core 1 β1,3-Gal-T1 (AF155582), which was registered by Ju et al., as a query, to find a gene comprising homology. Both a genome sequence (AC011890 (Xq23)) and cDNA sequences (AF150268 and BC011930) were registered for this gene, however, since these sequences showed only slight inconsistencies, cloning was carried out using a cDNA library. The sample used was the human colon cancer cell line Colo205 cDNA library, prepared by ordinary methods (Yuzuru Ikehara, Hisashi Narimatsu et al., Glycobiology Vol. 9, No. 11, pp. 1213-1224, 1999). In addition, an ordinary nucleic acid probe using a radioisotope was used for the screening methods.

First, PCR was carried out using primers CB-739 (5'-gaagatctag aatgcaccac catgagcatc-3'/ SEQ ID NO: 3) and CB-740 (5'-ataagaatgc ggccgctcag tcattgtcag aaccatttg-3'/ SEQ ID NO: 4), and using, as the template, λ phages prepared by ordinary methods from the human colon cancer cell line Colo205 cDNA library. The amplified 878-bp DNA fragment was then radioactively labeled with ³²P-dCTP using the Multiple DNA Labeling System (Amersham). A single λ phage plaque that hybridizes with this probe was selected from those formed on *E*. *coli*, and the presence of the subject DNA region was confirmed by PCR using the aforementioned primers CB-739 and CB-740. The phage, obtained from the plaque in which the DNA insertion was confirmed, was constructed with a λ ZAPII vector (Strategene) (Yuzuru Ikehara, Hisashi Narimatsu, et al., Glycobiology, Vol. 9, No. 11, pp. 1213-1224, 1999). Thus, it could be prepared as a cDNA clone that is inserted into pBluescript SK vector, of the methods described in the manual (Excision). This was prepared using the same methods, and a DNA was obtained from the resulting colonies. This cDNA clone was named SK-/C2. The 1471-bp nucleotide sequence of the cDNA clone was then identified of conventional methods (SEQ ID NO: 1; referred to as "Sequence 1"). A theoretical ORF (957 bp) was obtained, and from this 318 amino acids were predicted and given the name C1Gal-T2 (SEQ ID NO: 2) (Fig. 1). A 104-bp 5'-untranslated region and a 410-bp 3'-untranslated region were present, but poly(A) addition was not observed. Based on the amino acid sequence, this protein was predicted to be a typical type 2 membrane protein, observed in nearly all glycosyltransferases. A comparative study of Sequence 1 with the genome sequence registered as AC011890 revealed that one exon was comprised of the ORF, the 5-bp region upstream from ATG, and a 3'-untranslated region. It was further revealed that a 2746-bp intron was present between this exon and a 99-bp 5'-untranslated region. This exon-intron splicing site followed the GU-AG rule. Accordingly, C1Gal-T2 is composed of at least two exons. Although a cDNA sequence of nearly equal length was registered under Accession No. BC011930, a poly(A) was added at its 3' end. However, since this cDNA was 250 bp shorter than Sequence 1, excluding the poly(A) sequence, the presence of an mRNA isoform in which a poly(A) signal is selectively added to the 3'-untranslated region was suggested. The amino acid sequences of C1Gal-T1 and C1Gal-T2 were compared as queries, and despite homology of less than 30%, all seven cysteine residues were preserved. The DxD sequence, considered to be a bivalent cation bonding site, was present in both genes, but in different positions (Fig. 2).

### [Example 2] Incorporation of C1Gal-T2 into an expression vector

(a) In order to produce an expression system for C1Gal-T2, the entire ORF of C1Gal-T2 was incorporated into pDONR201 of the Gateway System (Invitrogen), and further recombined into pDEST12.2 (Invitrogen).
(b) Incorporation into pDONR201 using the Gateway system

### (i) Producting an entry clone

A DNA fragment was again obtained by PCR (15 cycles of 94°C for 15 seconds, 60°C for 30 seconds, and 68°C for one minute) using primers F (CB-760: 5'-ggggacaagt ttgtacaaaa aagcaggctt agaaggagat agaaccatgc tttctgaaag cagctcc-3'/ SEQ ID NO: 5) and R (CB-761: 5'-ggggaccact ttgtacaaga aagctgggtc tcaatcattgtcagaaccat-3'/ SEQ ID NO: 6), and using SK-/C2 as the template. The target fragment was cut out from the gel, purified, and then incorporated into pDONR201 through BP clonase reaction to produce an "entry clone". The reaction was carried out by incubating 5 µl of the target DNA fragment, 1 µl (150 ng) of pDONR201, 2 µl of reaction buffer, and 2 µl of BP clonase mix at 25°C for one hour. The reaction was terminated by adding 1 µl of Proteinase K and incubating at 37°C for ten minutes.

All of the aforementioned mixture (11 µl) was then mixed with 100 µl of competent cells (*E. coli* DH5α), subjected to heat shock treatment, and then plated onto an LB plate comprising kanamycin. The next day colonies were selected from the plate. The target DNA was directly confirmed using PCR, and the plasmid DNA (pDONR-C2) was extracted and purified.

### (ii) Production of expression clones

The aforementioned entry clone has a recombination site on both sides of the insertion site 'attL', for cleaving the λ phage from E. coli. Thus, by mixing this clone with LR clonase (a mixture of λ phase recombination enzymes Int, IHF, and Xis) and a destination vector, its insertion site migrates to the destination vector, resulting in an expression clone. The specific processes are described below:
First, 1 µl of entry clone, 0.5 µl (75 ng) of pDEST12.2, 2 µl of LR reaction buffer, 4.5 µl of TE, and 2 µl of LR clonase mix were reacted at 25°C for one hour. Then, 1 µl of Proteinase K was added thereto, and the mixture was incubated at 37°C for ten minutes to terminate the reaction (pDEST12.2-C2 is produced in this recombination reaction). pDEST12.2 is an animal cell expression vector comprising a neomycin-resistant gene commercially available from Invitrogen.

The entire volume of the aforementioned mixture (11 µl) was then mixed with 100 µl of competent cells (*E*. *coli* DH5α), subjected to heat shock treatment, and plated onto an LB plate comprising ampicillin. Colonies were selected from the plate the next day, the subject DNA was directly confirmed by PCR, and the vector (pDEST12.2-C2) was extracted and purified.

### [Example 3] Transfection of colon cancer cell line 'LSC' with pDEST12.2-C2

The colon cancer cell line LSC comprises GalNAc (Tn antigen: GalNAc-Ser/Thr) on a cell surface protein, but does not comprise core 1 synthesis activity (β1,3Gal-T activity on GalNAc). The expression of C1Gal-T2 was forced in this cell line by pDEST12.2-C2 transfection. Core 1 synthesis activity was detected by using the cell lysate as an enzyme source. The colon cancer cell line LSC was cultured in 10% fetal calf serum-RPMI-1640 medium (Invitrogen) (comprising streptomycin (100 µg/ml) /penicillin (100 units/ml) / L-glutamine (0.292 mg/ml)) at 37°C in the presence of 5% CO₂. The day before transfection the cells were plated (1.2x 10⁶ cells/2 ml) onto a 6-well dish. At this time, the medium was changed to that without streptomycin and penicillin. Transfection was carried out the day after plating. Ten µl of Lipofectamine 2000 (Invitrogen) was added to 250 µl of Opti-MEM (Invitrogen) and incubated at room temperature for five minutes. 250 µl of Opti-MEM comprising 10 µg of pDEST12.2-C2 was then mixed with the mixture and incubated at room temperature for 20 minutes. A total of 500 µl was dropped into the cells plated the previous day. Two days after transfection, the cells were separated from the container with trypsin (0.25%)-EDTA (1 mM) (Invitrogen). The cells were divided into two aliquots. One aliquot was again plated on to a new dish, while the other aliquot was washed twice with phosphate buffer and then stored at -80°C for measurement of activity during transient expression. The next day Geneticin (Invitrogen) was added to the re-plated cells to a final concentration of 0.6 mg/ml. This stably introduced cell line was named LSC-ClGal-T2.

### [Example 4] Screening for acceptor substrate of C1Gal-T2

Of about 100 types of known glycosyltransferase genes, C1Gal-T2 had the highest homology with core 1 Gal-T1, and was thus classified as a core 1 β1,3-galactosyltransferase. Thus the first study used UDP-Gal as a sugar donor substrate.

The reaction system below was used to investigate C2 acceptor substrates. To investigate whether pNp-α-GalNAc or pNp-β-GalNAc (both from Sigma) functioned as acceptors, each was used as an "acceptor substrate" in the following reaction solution:

The reaction solution (the parentheses show final concentrations) comprised acceptor substrate (10 nmol), HEPES buffer (pH 7.4) (14 mM), MnCl₂ (12.5 mM), UDP-Gal (250 µM), and UDP-[¹⁴C]-Gal (175 nCi). 5 µl of enzyme solution was added to this reaction solution, followed by the addition of H₂O to a total volume of 20 µl. The enzyme solution was prepared by suspending cells (5x 10⁶ cells) in 50 ml of cell lysing buffer (HEPES buffer (pH 7.4) (20 mM), NaCl (154 mM) and TritonX-100 (1%)), incubating at 4°C for 15 minutes in a water bath type ultrasonic homogenizer, centrifuging, and then obtaining the supernatant for the enzyme solution. The aforementioned reaction mixture was reacted at 37°C for two hours. Following completion of the reaction, 200 µl of H₂O was added to the mixture, which was gently centrifuged to obtain the supernatant. The supernatant was passed through a Sep-Pak plus C18 Cartridge (Waters) previously equilibrated by washing once with 10 ml of methanol, and then twice with 10 ml of H₂O. The substrate and product in the supernatant were adsorbed to the cartridge. After washing the cartridge twice with 10 ml of H₂O, the adsorbed substrate and product were eluted with 5 ml of methanol. The eluate was dried to a solid while blown with nitrogen gas and heated with a 40°C heating block. 20 µl of methanol was added to the resulting solid, blotted onto a TLC plate (HPTLC plate Silica gel 60: Merck), and then developed using a developing solvent (chloroform:methanol:water(comprising 0.2% CaCl₂) = 55:45:8). The solution was developed to 5 mm from the upper edge of the TLC plate. After drying the plate, the amount of radioactivity taken up by the product was measured using the Bio Image Analyzer FLA3000 (Fuji Photo Film).

The results showed that the LSC-C1Gal-T2 cell extract reacted strongly to pNp-α-GalNAc, but did not react to pNp-β-GalNAc. Thus, C1Gal-T2 was suggested to be a synthetase of galactose β1-3-N-acetylgalactosaminyl α1-R (Fig. 3).

### [Example 5] Confirmation of activity using N-acetylgalactosaminyl-peptides (GalNAc α1-peptides) as acceptor substrates

Since the above-described Example suggested that C1Gal-T2 was a synthetase of core 1 sugar chain (galactose β1-3N-acetylgalactosaminyl α1-R) synthetase, the above experiment was repeated using various GalNAc α1-peptides as acceptor substrates. This resulted in similar reactions to that with pNp-α-GalNAc.

In order to investigate galactosyltransferase activity on GalNAc α1-peptide acceptor substrates, acceptor substrates were prepared of the following method:
Peptides were synthesized in which a single GalNAc was introduced into the -OH group of the 4th, 7th, 9th, 11th or 15th S or T residue of the peptide sequence HP (VPSTPPTPSPSTPPTPSPS/SEQ ID NO: 7), which is in the hinge region of human IgAl. An additional peptide was synthesized whereby GalNAc was introduced into each of the -OH groups of the 4th, 7th, 9th, 11th and 15th S or T residues (Peptide Institute). The synthesized peptides were named 4-GalNAc-HP (VPST(GalNAc)PPTPSPSTPPTPSPS), 7-GalNAc-HP (VPSTPPT(GalNAc)PSPSTPPTPSPS), 9-GalNAc-HP (VPSTPPTPS(GalNAc)PSTPPTPSPS), 11-GalNAc-HP (VPSTPPTPSPS(GalNAc)TPPTPSPS), 15-GalNAc-HP (VPSTPPTPSPSTPPT(GalNAc)PSPS), and 4,7,9,11,15-GalNAc-HP (VPST(GalNAc)PPT(GalNAc)PS(GalNAc)PS(GalNAc)TPPT(GalNAc)PSPS). Each type of GalNAc-HP serving as an acceptor substrate was dissolved in H₂O, mixed with Cy5 in dimethylformamide to a molar ratio of 1:10, and then subjected to an overnight Cy5-labeling reaction at 4°C. The reaction solution was purified by high-performance liquid chromatography (HPLC). Specifically, CAPCELL PAK C₁₈ UG120 (Shiseido) was used as the column, 0.1% trifluoroacetic acid was used as the separation buffer, the elution was carried out over a 15-30% acetonitrile concentration gradient, the flow rate was 1 ml/minute, and the conditions detected were excitation wavelength at 649 nm and fluorescence wavelength at 670 nm. Using the fluorescence of Cy5 as an indicator, substrates were separated as single substrate peaks at the retention times of 35.2, 34.4, 35.1, 34.9, 34.6, and 31.1 minutes for each type of Cy5-labeled GalNAc-HP (4-GalNAc-HP-Cy5, 7-GalNAc-HP-Cy5, 9-GalNAc-HP-Cy5, 11-GalNAc-HP-Cy5, 15-GalNAc-HP-Cy5, and 4,7,9,11,15-GalNAc-HP-Cy5 respectively). The separated substrates were concentrated by freeze-drying. The substrates prepared in this manner were named 4-GalNAc-HP-Cy5, 7-GalNAc-HP-Cy5, 9-GalNAc-HP-Cy5, 11-GalNAc-HP-Cy5, 15-GalNAc-HP-Cy5, and 4,7,9,11,15-GalNAc-HP-Cy5.

FITC-labeled and human digestive organ-derived mucin peptide sequence FITC-MUC1a' (FITC-AHGVTSAPDTR) and, FITC-labeled and rat submandibular gland-derived mucin peptide sequence EA2-FITC (PTTDSTTPAPTTK-FITC) were synthesized (Sawaday Technology). Moreover, resulting FITC-MUC1a' or EA2-FITC peptide as an acceptor substrate was reacted with a known UDP-N-acetyl-D-galactosamine: polypeptide N-acetylgalactosaminyltransferase (GalNAc-T6 or GalNAc-T10). The reaction solution was analyzed by HPLC. Specifically, 5C₁₈-AR Code No. 378-66 (COSMOSIL) was used as the column, 0.05% trifluoroacetic acid was used as the separation buffer, the elution was carried out over a 0-50% acetonitrile concentration gradient, the flow rate was 1 ml/minute, and the detected conditions were excitation wavelength at 492 nm and fluorescence wavelength at 520 nm. A single product peak (retention time: 18.8 minutes) indicating a difference of 0.8 minutes from the peak of acceptor substrate FITC-MUC1a' (retention time: 19.6 minutes) was separated, and a single product peak (retention time: 19.6 minutes) indicating a difference of 0.7 minutes from the acceptor substrate EA2-FITC peak (retention time: 20.3 minutes) was separated. After freeze-drying, portions were analyzed using MALDI-mass spectrometry (REFLEX, BRUKER), and both products were confirmed to comprise a single GalNAc transferred to the acceptor substrate. The substrates prepared in this manner were named FITC-MUC1a'-GalNAc and EA2-GalNAc-FITC.

The reaction solution (parentheses indicate final concentrations) comprised acceptor substrate (5 pmol), MES buffer (pH 6.5) (100 mM) , MnCl₂ (20 mM), ATP (2 mM), and UDP-Gal (0.5 mM). 5 µl of LSC-C1Gal-T2 enzyme source was added to this reaction solution, which was then brought to a total volume of 20 µl by adding H₂O.

The above reaction mixture was reacted at 37°C for eight hours. After completion of the reaction, 60 µl of H₂O was added, and the mixture was gently centrifuged to acquire the supernatant. This supernatant was purified through an Ultra Free MC Column (Millipore), and 30 µl of thus-purified solution was analyzed using high-performance liquid chromatography (HPLC). CAPCELL PAK C₁₈ UG120 (Shiseido) was used as the column, 0.1% trifluoroacetic acid was used as the separation buffer, and the elution was carried out over an acetonitrile concentration gradient of 15-30%. The flow rate was 1 ml/minute.

When using the enzyme source prepared with the aforementioned cell lysing buffer, peptides of the acceptor substrate, GalNAc α1-peptide, are degraded, and thus HPLC analysis detects a large number of peaks. Accordingly, when using GalNAc α1-peptide as the acceptor substrate, a microsome fraction was prepared from an LSC-C1Gal-T2 transfectant and used as the enzyme source. In this case, peptide degradation can be suppressed, and HPLC can detect the target product as a single peak.

The method for preparing the microsome fraction is detailed below:
A cell line of 1x 10⁸ cells was suspended in 1 ml of 0.25 M sucrose/10 mM Tris-HCl (pH 7.4) followed by the addition of 100 µl of Protease Inhibitor Cocktail (Sigma). The cell suspension was transferred to a Dounce tissue homogenizer and the cells were homogenized with 200 strokes. The supernatant was then recovered by centrifuging at 1000 x g for ten minutes at 4°C. The supernatant was further centrifuged at 105,000 x g for one hour at 4°C to obtain the precipitate as a microsome fraction, which was then dissolved in a suitable amount of 0.25 M sucrose/10 mM Tris-HCl (pH 7.4) and used as the enzyme source.

As a result, when using 11-GalNAc-HP-Cy5 as the acceptor substrate and UDP-Gal as the donor substrate, the reaction product appeared as a new peak at 28.8 minutes (Figs. 4A and 4B).

### [Example 6] Analysis of binding manner of galactose and N-acetylgalactosaminyl α1-R

The binding manner employed between galactose and N-acetylgalactosamine was confirmed by glycosidase treatment. A reaction product in which galactose was transferred to 11-GalNAc-HP-Cy5 by LSC-C1Gal-T2 was treated with β1,3-galactosidase (Meiji Dairies). Since the peak of the 11-GalNAc-HP-Cy5 in which the galactose was transferred was confirmed by HPLC to migrate to the original peak of 11-GalNAc-HP-Cy5, C1Gal-T2 was verified *in vitro* to be a core 1 synthetase, by which galactose is transferred to the nonreducing terminal of GalNAc α1-peptide through the β1,3 bond (Fig. 4C).

### [Example 7] Analysis of the sugar chain structure of glycoproteins on the surface of cultured cells

Core 1 β1,3 galactosyltransferase activity is not detected in the colon cancer cell line LSC. Accordingly, expression of Tn antigen and sialyl Tn antigen is observed on the surfaces of these cells. A stable C1Gal-T2 transfectant (LSC-C1Gal-T2) was produced using this cell line. Changes in cell surface sugar chain antigens were then measured using a flow cytometer. The LSC-C1Gal-T2 production method has already been described. The cells were separated from the dish using trypsin (0.25%) - EDTA (1 mM), and washed with 0.1% bovine serum albumin / 0.1% azide /phosphate buffer. 1x 10⁵ cells were stained with FITC-labeled peanut agglutinin rectin (PNA-FITC: EY Laboratories, Inc.), HB STn1 (anti-sialyl Tn antigen monoclonal antibody, mouse IgM: Dako), and HB-T1 (anti-Tn antigen monoclonal antibody, mouse IgGl: Dako), which recognize the core 1 structure. 50 µg/ml PNA-FITC, and x100-diluted HB STn1 and HB-T1 were reacted with the cells at 4°C for 30 minutes. The cells were washed twice with 0.1% bovine serum albumin / 0.1% azide / phosphate buffer. FITC-labeled secondary antibodies (anti-mouse IgG and IgM antibodies both diluted 1000-fold) were then reacted at 4°C for 30 minutes with cells stained with HB STn1 and HB-T1. The cells were washed twice with 0.1% bovine serum albumin / 0.1% azide / phosphate buffer. The cells were then fixed with 0.5% paraformaldehyde /phosphate buffer, and FITC intensity was measured using FACSCaliber (Becton-Dickinson). Compared to LSC cells, LSC-C1Gal-T2 demonstrated increased PNA staining, and decreased Tn and sialyl Tn antigen staining (Fig. 5). Tn antigens on LSC cell surfaces became T antigens when galactose was transferred by the action of C1Gal-T2. In addition, sialyl Tn antigen decreased since C1Gal-T2 won the competition with endogenous sialyltransferase. This caused expression of the T antigen. On the basis of these results, C1Gal-T2 was also demonstrated to be an intracellular core 1 synthetase.

### [Example 8] Expression in various human tissues and established cells

The expressed amounts of cDNAs derived from normal human tissues and established cells were quantified using quantitative PCR. Marathon Ready cDNA (Clontech) was used as the normal tissue cDNA. For the established cell lines, total RNA was extracted and cDNA was then produced by ordinary methods. The primers used for quantitative expression analysis of C1Gal-T2 were C2-RT-FP1 (5'-gtttgcctga aatatgctgg agtat-3'/ SEQ ID NO: 8) and C2-RT-RP3 (5'-caacagcctt ctactacctg gttg-3'/ SEQ ID NO: 9). C2-RT-MGB1 (5'-cagaaaatgc agaagatgct gatggaaaag atgta-3'/ SEQ ID NO: 10) was used as the probe. Furthermore, the C2-RT-MGB1 probe was used was bound to Minor Group Binder (Applied Biosystems). The enzyme and reaction solution both used Universal PCR Master Mix, and quantification was carried out using 25 µl of reaction solution, with the ABI PRISM 7700 Sequence Detection System (both Applied Biosystems). The glyceraldehyde-3-phosphate dehydrogenase (GAPDH) gene was used as the quantification standard, and the amount of gene expression was standardized based on a quantitative standard curve using known concentrations of a template DNA. The reaction was carried out for two minutes at 50°C, ten minutes at 95°C, and 50 cycles of 15 seconds at 95°C and one minute at 60°C. These results are shown in Fig. 6.

### [Example 9] Analysis of C1Gal-T2 Variants

Human cell lines, LSC and Jurkat cells are known to lack core 1 synthesis activity. Real-time PCR was used to quantify C1Gal-T1 and C1Gal-T2 gene expression in these two cell lines. They were thus confirmed to have expression levels equal to those of cell lines LSB and K562 cells, which comprise core 1 synthesis activity (Fig. 8).

The C1Gal-T1 and C1Gal-T2 genes were amplified by PCR from the genomic DNA and cDNA of these four cell lines, and their nucleotide sequences were determined by direct sequencing. Primers 5'-AGAAATACACTTTTCGGGAA-3' (SEQ ID NO: 11) and 5'-TGCAGTGCTAGACATATTAC-3' (SEQ ID NO: 12) were used as amplification primers for the cDNA of C1Gal-T1. Primers 5'-GCTTTCCTGTCCCCAAGCCGTTC-3' (SEQ ID NO: 13) and 5'-GCCCCACAGCTTTCTAATGTTC-3' (SEQ ID NO: 14) were used as amplification primers for the cDNA of C1Gal-T2. In addition, primers 5'-GTAATCAGATTCCATTGGAAGC-3' (SEQ ID NO: 15) and 5'-GCCCCACAGATTTCTAATGTTC-3' (SEQ ID NO: 14) were used as amplification primers for the genomic DNA of ClGal-T2.

The C1Gal-T1 sequence was identical in all of the cDNA and genomic DNA sequences registered for the four cell lines. The C1Gal-T2 sequence of the LSB and K562 cells comprising core 1 synthesis activity was identical to the sequence whose cDNA and genomic DNA were both confirmed to comprise core 1 synthesis activity (Fig. 9A). In the C1Gal-T2 sequence of the LSC cells, one residue T was inserted between the 53rd T and 54th C residues, measured from A of the ATG starting codon (Fig. 9B). As a result, a frame shift occurred and the stop codon appeared at an intermediate location. The C1Gal-T2 sequence in the Jurkat cells comprised an alternation of the 428th C to a T, resulting in a missense variation of the amino acid, from alanine to valine, and a deletion of the 468th T (Fig. 9C). A frame shift also occurred in this case, and the stop codon appeared at an intermediate location.

The C1Gal-T2 gene is located on the X chromosome, and has one allele in males, or two alleles in females. Jurkat cells are male-derived, and comprise an inactive C1Gal-T2 on the single X chromosome. The only LSC cells found comprised either a cDNA or genomic ClGal-T2 sequence mutation, although it was not possible to identify which.

As described above, since C1Gal-T2 is inactive, LSC and Jurkat cells were not found to comprise core 1 synthesis activity. In addition, since C1Gal-T1 core 1 synthetase activity could not be detected even though mRNA was expressed, the specific activity of ClGal-T2 was suggested to be even more potent.

### [Example 10] Identification of Novel Galactosyltransferase C1Gal-T3

Public databases were searched using the C1Gal-T2 (AB084170) identified by the present inventors as a query, and a sequence comprising homology was recorded. In humans this gene only exists in the form of genomic sequence information (AC011242 and AC084264 (the second chromosome)), and is predicted to comprise an ORF of a single exon. It is 68% homologous to C1Gal-T2 at the amino acid level, and comprises six of the seven conserved cysteine residues (Fig. 10). The present inventors named the novel galactosyltransferase encoded by this sequence 'C1Gal-T3'. In addition, a clone 96% homologous to this gene was registered as a Macaca fascicularis testis cDNA clone (AB071109).

### [Example 11] Incorporation of C1Gal-T3 into an expression vector

(a) To produce an expression system for C1Gal-T3, the entire ORF of C1Gal-T3 was incorporated into pDONR201 of the Gateway System (Invitrogen), and then re-introduced into pDEST12.2 (Invitrogen).
(b) Incorporation into pDONR201 using the Gateway System was performed as described below:

### (i) Preparating an entry clone

A DNA fragment was obtained by PCR (30 cycles of ten seconds at 98°C, 30 seconds at 55°C, and 90 seconds at 78°C) using primers F (C5GFSKD1: 5'-ggggacaagt ttgtacaaaa aagcaggctt cgaaggagat agaaccatgg tttccgctag tgggacatc-3'/ SEQ ID NO: 16) and R (C5GR: 5'-ggggaccact ttgtacaaga aagctgggtc tcagtcattt tctgaaccaa ctggag-3'/ SEQ ID NO: 17), and using uterus-derived cDNA (Marathon-Ready cDNA, Clontech) as the template. The target fragment was cut out from the gel, purified, and incorporated into pDONR201 by a BP clonase reaction, producing an "entry clone". The reaction was carried out by incubating 2 µl of the target DNA fragment, 1 µl (150 ng) of pDONR201, 2 µl of BP reaction buffer, and 2 µl of BP clonase mix, at 25°C for one hour. The reaction was terminated by adding 1 µl of Proteinase K and incubating at 37°C for ten minutes. One µl of the reaction mixture was mixed with 50 µl of competent cells (*E. coli* DH5α), the mixture was subjected to heat shock treatment, and then plated onto an LB plate comprising kanamycin. The colonies were selected from the plate on the following day, the target DNA was directly confirmed using PCR, and the plasmid DNA (pDONR-C1Gal-T3) was extracted and purified. A theoretical 948-bp ORF (SEQ ID NO: 18) was obtained, and 315 amino acids (SEQ ID NO: 19) were thus predicted from this ORF. Based on the amino acid sequence, this protein was predicted to be a typical type 2 membrane protein, observed in most glycosyltransferases.

### (ii) Production of expression clone

First, 1 µl of entry clone, 1 µl (150 ng) of pDEST12.2, 2 µl of LR reaction buffer, 4 µl of TE, and 2 µl of LR clonase mix were reacted at 25°C for one hour. One µl of Proteinase K was added to the mixture, which was then incubated at 37°C for ten minutes to terminate the reaction. pDEST12.2 is an animal cell expression vector comprising a neomycin-resistant gene (commercially available from Invitrogen).

Subsequently, 1 µl of the aforementioned reaction mixture was mixed with 50 µl of competent cells (*E. coli* DH5α), the mixture was subjected to heat shock treatment, and then plated onto an LB plate comprising ampicillin. The colonies were picked up from the plate on the following day, the subject DNA was confirmed directly by PCR, and the vector (pDEST12.2-C1Gal-T3) was extracted and purified.

### [Example 12] Incorporation of FLAG tagged C1Gal-T3 fusion protein expression vector

DNA fragments were obtained by PCR (18 cycles of ten seconds at 98°C, 30 seconds at 55°C, and 90 seconds at 72°C) using primers F (TKC-7: 5'-gccccaagctt cacagaggtc aaactcaaga ccac-3' (SEQ ID NO: 20/ underlined region indicates cleavage sequence for HindIII) and R (TCK-9: 5'-cggaattctc agtcattttc tgaaccaact g-3' (SEQ ID NO: 21/ underlined region indicates cleavage sequence for EcoRI), and using pDONR-C1Gal-T3 DNA as the template. After restriction enzyme treatment (HindIII-EcoRI), the target fragment was cut out from the gel, purified, and incorporated into pFLAG-CMV-3 vector (Sigma) via a DNA ligase reaction, to obtain pFLAG-ClGa1-T3. From its 5' side the pFLAG-CMV-3 vector comprises a preprotrypsin secretion signal, and a FLAG sequence as a tag. Recombinant proteins can be prepared by inserting a target gene to the 3' of these sequences. For glycosyltransferase genes, it is known that proteins comprising glycosyltransferase activity can be obtained by introducing a carboxyl terminal region (enzyme activity region) minus the transmembrane region at the amino terminus.

### [Example 13] Transient transfection of pFLAG-C1Gal-T3 into COS-1 cells

Large amounts of plasmid pFLAG-C1Gal-T3 DNA were purified using the CONCERT High Purity Plasmid Maxiprep System (Invitrogen). COS cells were cultured in 10% fetal calf serum-DMEM medium (Invitrogen) (comprising streptomycin (100 µg/ml) and penicillin (100 units/ml)) at 37°C in the presence of 5% CO₂. The day before transfection, cells were plated at 3x 10⁶ cells/12 ml onto a 9-cm dish. At this time, the medium was exchanged to that without streptomycin and penicillin. Transfection was carried out the day after plating. 60 µl of Lipofectamine 2000 (Invitrogen) was added to 1.5 ml of Opti-MEM (Invitrogen) and incubated at room temperature for five minutes. 30 µg of pFLAG-C1Gal-T3 in 1.5 ml of Opti-MEM was then mixed, and this mixture was incubated at room temperature for 20 minutes. A total of 3 ml of the resulting mixture was dropped into the cells plated on the previous day. The culture supernatant was recovered two days after transfection and stored at -80°C until the time of use.

### [Example 14] Purification of recombinant enzyme from the culture supernatant, and confirmation of the same

100 µl of anti-FLAG M1 monoclonal antibody-agarose affinity gel, 2 mM CaCl₂, 150 mM NaCl₂, and 0.05% NaN₃ were added to 10 ml of culture supernatant. Adsorption was carried out at 4°C overnight while rotating on a rotating plate such that the resin was mixed. This was twice washed with 1 mM CaCl₂/TBS buffer (50 mM Tris-HCl (pH 7.4) and 150 mM NaCl), and 50 µl of extraction buffer (2 mM EDTA/TBS buffer) was then added thereto. A portion of this mixture was then subject to SDS-polyacrylamide electrophoresis. The resulting proteins were transferred to a Hybond-P Nylon membrane, and Western blotting analysis was carried out using anti-FLAG M1 monoclonal antibody (Sigma). C1Gal-T3 recombinant enzyme could be detected at about 40 kDa using Konica Immunostain HRP1000 coloring (Konica).

### [Example 15] Confirmation of C1Gal-T3 activity using N-acetylgalactosaminyl peptides (GalNAcα1-peptides) as acceptor substrates

Galactose transferring activity was measured using the C1Gal-T3 recombinant enzyme expressed in COS-1 cells as an enzyme source, and using the N-acetylgalactosaminyl peptides used in Example 5 as acceptor substrates. As a result, galactose transferring activity could be confirmed for 4-GalNAc-HP.

### [Example 16] Expression of C1Gal-T3 Transcription Product in Various Human Tissues

The amount of gene expression was quantified by quantitative PCR using normal human tissue. cDNAs in the normal tissue were prepared from total RNA (Clontech) in accordance with conventional methods. The primers used for quantitative expression analysis of C1Gal-T3 were C5-RT-FP1 (5'-gcctgaaata tgcaggagtt ca-3'/ SEQ ID NO: 22) and C5-RT-RP2 (5'-ggttattaga caatgcctct tcaataag-3'/ SEQ ID NO: 23). C5-RT-MGB1 (5'-FAM-gcagaaaatg cagaggatta tgaaggaaga gatgta-MGB-TAMRA-3'/ SEQ ID NO: 24) was used as the probe. The C5-RT-MGB1 probe was bound to the Minor Group Binder (Applied Biosystems). The enzyme and reaction solutions used Universal PCR Master Mix, and quantification was carried out using the ABI PRISM 7700 Sequence Detection System and 25 µl of reaction solution (both Applied Biosystems). The glyceraldehyde-3-phosphate dehydrogenase (GAPDH) gene was used as the quantification standard, and the amount of gene expression was standardized based on a quantitative standard curve, using known concentrations of a template DNA. The reaction was carried for two minutes at 50°C, ten minutes at 95°C, and 50 cycles of 15 seconds at 95°C and one minute at 60°C. These results are shown in Fig. 11.

### [Example 17] Expression of C1Gal-T1, -T2, and -T3 transcription products in various human tissues, using real-time PCR

Quantitative analyses were carried out on human C1Gal-T3 transcription products in fractions of human peripheral blood mononuclear cells, B cells, helper T cells, and killer T cells, using real-time PCR. Using Ficol, peripheral blood mononuclear cells were separated from whole blood obtained from healthy volunteers. The B cell, helper T cell, and killer T cell fractions were obtained from peripheral blood mononuclear cells using beads to which anti-CD19, anti-CD4, and anti-CD8 antibodies were respectively bound. C1Gal-T3 and GAPDH standard curves were generated from a dilution series of each plasmid. The expression level of C1Gal-T3 transcription product was standardized using the expression level of GADPH transcription product. As a control, expression in the testis, in which C1Gal-T3 expression is relatively high, was also measured at the same time (Fig. 12).

In addition, quantitative analyses of the C1Gal-T1, -T2, and -T3 transcription products in blood cells were also carried out in the same manner as for the aforementioned C1Gal-T3. These three were then compared (Fig. 13).

Quantitative analysis of C1Gal-T3 transcription products was also carried out by real-time PCR, using various cell lines derived from human B cells (Fig. 14). Expression of C1Gal-T3 was observed in B cells. In addition, quantitative analyses for the transcription products of C1Gal-T1, -T2, and -T3 in various cell lines derived from human B cells were also carried out using real-time PCR, and these three were then compared (Fig. 15).

### [Example 18] Transfection of C1Gal-T3 into COS-1 cells

ClGal-T3 was transfected into COS-1 cells. pFLAG-CMV3-C1Gal-T3 was purified from the culture supernatant of the transfected COS-1 cells using M1 agarose bound with anti-FLAG antibody. 12.5% acrylamide gel was used. The results of SDS-PAGE and Coomassie staining of C1Gal-T3 are shown in Fig. 16.

Moreover, the core 1 synthesis activities of the COS-1-ClGal-T3 transfectant on GalNAc-peptide were also analyzed. Using C1Gal-T3 purified from the culture supernatant, core 1 synthesis reactions were carried out using fluorescent-labeled N-acetylgalactosamine α1-O-Serine (GalNAc-Ser) or GalNAc-peptide as the acceptor substrate, and using UDP-Gal as the donor substrate. These reactions were then analyzed by HPLC.

Fig. 17 shows the results of using, as the enzyme sources, i) C1Gal-T3 purified from the COS-1-pFLAG-CMV3-C1Gal-T3 transfectant culture supernatant using the FLAG tag, ii) cell extracts of cell line LSB comprising core 1 synthesis activity (positive control), and iii) cell extracts of a cell line LSC that does not comprise core 1 synthesis activity (negative control). Fluorescent-labeled GalNAc-Ser was used as the acceptor.

Fig. 18 shows the results for all enzyme sources of reactions using C1Gal-T3 purified from the COS-1-pFLAG-CMV3-C1Gal-T3 transfectant culture supernatant using FLAG tag.

### [Example 19] Comparison of substrate specificity of C1Gal-T1, -T2, and -T3

The substrate specificities of C1Gal-T1, -T2, and -T3 were compared. Microsome fractions of LSC-C1Gal-T1 and LSC-C1GalT2 and purified enzyme derived from COS-1-C1Gal-T3 were used as enzyme sources to analyze core 1 synthesis activity for each acceptor substrate (Fig. 18).

### [Example 20] Co-Expression of C1Gal-T1, -T2, and -T3

C1Gal-T1, -T2, and -T3 were co-expressed in 293T cells in the combinations indicated below:
1 pFLAG-CMV3-C1Gal-T1 + pCDNAIHN
2 pFLAG-CMV3-C1Gal-T1 + pCDNAIHN-C1Gal-T1
3 pFLAG-CMV3-C1Gal-T1 + pCDNAIHN-C1Gal-T2
4 pFLAG-CMV3-C1Gal-T1 + pCDNAIHN-C1Gal-T3
5 pFLAG-CMV3-C1Gal-T1 + pCLN
6 pFLAG-CMV3-C1Gal-T1 + pCLN-C1Gal-T1
7 pFLAG-CMV3-C1Gal-T1 + pCLN-C1Gal-T2
8 pFLAG-CMV3-C1Gal-T1 + pCLN-C1Gal-T3
9 pFLAG-CMV3-C1Gal-T2 + pCLN
10 pFLAG-CMV3-C1Gal-T2 + pCLN-C1Gal-T1
11 pFLAG-CMV3-C1Gal-T3 + pCLN
12 pFLAG-CMV3-C1Gal-T3 + pCLN-C1Gal-T1

To analyze co-expression, Western blotting was carried out with x1000 anti-FLAG antibodies for C1Gal-T1, -T2, and -T3 purified from culture supernatant and cells (Fig. 19).

Moreover, core 1 synthesis activity was analyzed using 293T transfectants co-expressing C1Gal-T1, -T2, and -T3. Core 1 synthesis activity on GalNAc-peptide was analyzed by HPLC using C1Gal-T purified from the culture supernatant of each 293T transfectant (Fig. 20). IgA hinge peptides that bind to specific GalNAc were used as acceptor substrates.

### [Example 21] Preparation of C1Gal-T2 Disease Model Mice

Knockout mice were obtained as follows: First, 80 µg of a linear targeting vector, inserted with an approximately 10 kb chromosome fragment comprising the entire ORF of mouse C1Gal-T2, was transfected (by electroporation) into ES cells (derived from E14/129Sv mice) to select G418-resistant colonies. G418-resistant colonies were transferred to a 24-well plate and cultured. After freezing a portion of the cells for storage, DNAs were extracted from the remaining ES cells. About 120 colonies of clones in which recombination occurred due to PCR were selected. After using Southern blotting to confirm that recombination had occurred as planned, about ten clones were finally selected as recombinants. ES cells from two of the selected clones were injected into the blastocycts of C57BL/6 mice. The mouse embryos to which ES cells were injected were transplanted into the uterus of a surrogate mother to generate chimeric mice. Hetero-knockout mice were then obtained by germ transmission.

Although a mouse homolog of C1Gal-T3 has yet to be found, knockdown or transgenic mice can be obtained by siRNA methods in human-derived cells.

### Industrial Applicability

The present invention provides polynucleotides that encode novel galactosyltransferases, vectors that comprise these polynucleotides, host cells comprising these vectors, polypeptides encoded by these polynucleotides, and methods for producing these polypeptides. Moreover, methods for identifying compounds that change the galactose transferring activity of the polypeptides are also provided. The polypeptides or polynucleotides of the present invention, or the compounds that change the galactose transferring activities of the polypeptides of the present invention, are expected to be used for the development of novel preventive and therapeutic agents for diseases related to polypeptides of the present invention. Moreover, the present invention also provides disease testing methods that comprise detecting mutations and expression of genes that encode the polypeptides of the present invention. Galactosyltransferase is one of the most important molecules attracting attention in the field of pharmaceutical development and health care. These fields are expected to progress thanks to the novel galactosyltransferase provided by the present invention. The present invention is also expected to be a valuable source of information for researchers of galactosyltransferase.

## Claims

1. A polynucleotide selected from the group consisting of:
(a) a polynucleotide that encodes a polypeptide comprising the amino acid sequence of SEQ ID NO: 2 or 19; and
(b) a polynucleotide that comprises a coding region of the nucleotide sequence of SEQ ID NO: 1 or 18.

2. A polynucleotide comprising galactose transferring activity, selected from the group consisting of:
(c) a polynucleotide that encodes a polypeptide comprising the amino acid sequence of SEQ ID NO: 2 or 19, wherein one or more amino acids are substituted, deleted, added, and/or inserted; and
(d) a polynucleotide that hybridizes with a DNA comprising the nucleotide sequence of SEQ ID NO: 1 or 18 under stringent conditions.

3. A polynucleotide that encodes a fragment of a polypeptide comprising the amino acid sequence of SEQ ID NO: 2 or 19.

4. A vector that comprises the polynucleotide of any one of claims 1 to 3.

5. A host cell that comprises the polynucleotide of any one of claims 1 to 3 or the vector of claim 4.

6. A polypeptide encoded by the polynucleotide of any one of claims 1 to 3.

7. A method for producing the polypeptide of claim 6, which comprises the steps of:
culturing the host cell of claim 5; and
recovering the polypeptide produced from the host cell or the culture supernatant of the same.

8. An antibody that binds to the polypeptide of claim 6.

9. A pharmaceutical composition for treating a patient who requires an increase in the activity or expression of the polypeptide of claim 6, wherein the composition comprises a therapeutically effective amount of a molecule selected from the group consisting of:
(a) the polynucleotide of any one of claims 1 to 3;
(b) the vector of claim 4; and
(c) the polypeptide of claim 6.

10. A pharmaceutical composition for treating a patient who requires suppression of the activity or expression of the polypeptide of claim 6, wherein the composition comprises a therapeutically effective amount of a molecule selected from the group consisting of:
(a) the antibody of claim 8; and
(b) a polynucleotide that suppresses the expression of an endogenous gene encoding the polypeptide of claim 6 *in vivo.*

11. A method of screening for a candidate therapeutic compound for a disease related to abnormal expression of a gene encoding the polypeptide of claim 6, or abnormal activity of the polypeptide of claim 6, which comprises the steps of:
(a) contacting a test compound with the polypeptide of claim 6;
(b) measuring the galactose transferring activity of the polypeptide of claim 6; and
(c) selecting a compound that changes the galactose transferring activity, compared to when the test compound is not contacted.

12. A method of testing for a disease related to abnormal expression of a gene encoding the polypeptide of claim 6, or abnormal activity of the polypeptide of claim 6, which comprises the step of detecting a mutation in the gene or its expression control region, in a patient.

13. The method of claim 12, which comprises the steps of:
(a) preparing a DNA sample from a subject;
(b) isolating a DNA that encodes the polypeptide of claim 6 or its expression control region;
(c) determining the nucleotide sequence of the isolated DNA; and
(d) comparing the nucleotide sequence of the DNA of step (c) with that of a control.

14. The method of claim 12, which comprises the steps of:
(a) preparing a DNA sample from a subject;
(b) cleaving the prepared DNA sample with a restriction enzyme;
(c) separating the DNA fragments by size; and
(d) comparing the size of the detected DNA fragments with that of a control.

15. The method of claim 12, which comprises the steps of:
(a) preparing a DNA sample from a subject;
(b) amplifying a DNA that encodes the polypeptide of claim 6 or its expression control region;
(c) cleaving the amplified DNA with a restriction enzyme;
(d) separating the DNA fragments by size; and
(e) comparing the size of the detected DNA fragments with that of a control.

16. The method of claim 12, which comprises the steps of:
(a) preparing a DNA sample from a subject;
(b) amplifying a DNA that encodes the polypeptide of claim 6 or its expression control region;
(c) dissociating the amplified DNA into a single strand DNA;
(d) separating the dissociated single strand DNA on non-denaturing gel; and
(e) comparing the mobility of the separated DNA on the gel with that of a control.

17. The method of claim 12, which comprises the steps of:
(a) preparing a DNA sample from a subject;
(b) amplifying a DNA that encodes the polypeptide of claim 6 or its expression control region;
(c) separating the amplified DNA on a gel that comprises a gradually increasing concentration of a DNA denaturant; and
(d) comparing the mobility of the separated DNA on the gel with that of a control.

18. A method of testing for a disease related to abnormal expression of a gene encoding the polypeptide of claim 6, which comprises the step of detecting the expression level of the gene in a subject.

19. The method of claim 18, which comprises the steps of:
(a) preparing an RNA sample from a subject;
(b) measuring the amount of RNA that encodes the polypeptide of claim 6 comprised in the RNA sample; and
(c) comparing the measured amount of RNA with that of a control.

20. The method of claim 18, which comprises the steps of:
(a) providing a cDNA sample prepared from a subject, and a board on which a nucleotide probe that hybridizes with a DNA encoding the polypeptide of claim 6 is immobilized;
(b) contacting the cDNA sample with the board;
(c) measuring the expression level of a gene encoding the polypeptide of claim 6 comprised in the cDNA sample, by detecting the intensity of hybridization between the cDNA sample and the nucleotide probe immobilized on the board; and
(d) comparing the measured expression level of the gene encoding the polypeptide of claim 6 with that in a control.

21. The method of claim 18, which comprises the steps of:
(a) preparing a protein sample from a subject;
(b) measuring the amount of the polypeptide of claim 6 comprised in the protein sample; and
(c) comparing the measured amount of the polypeptide with that of a control.

22. The method of any one of claims 12 to 21, wherein the disease is IgA nephropathy or Tn syndrome.

23. An oligonucleotide comprising at least 15 nucleotides that hybridizes with a DNA encoding the polypeptide of claim 6 or an expression control region thereof.

24. A drug comprising the oligonucleotide of claim 23, for testing for a disease related to abnormal expression of a gene encoding the polypeptide of claim 6, or abnormal activity of the polypeptide of claim 6.

25. A pharmaceutical comprising the antibody of claim 8, for testing for a disease related to abnormal expression of a gene encoding the polypeptide of claim 6, or abnormal activity of the polypeptide of claim 6.

26. The pharmaceutical of claim 24 or 25, wherein the disease is IgA nephropathy or Tn syndrome.

27. A genetically altered non-human animal wherein the expression of C1Gal-T2 protein is artificially altered.

28. A genetically altered non-human animal into which an exogenous polynucleotide that encodes ClGal-T2 protein has been introduced.

29. The genetically altered non-human animal of claim 27 or 28, wherein the non-human animal is a mouse.

30. A cell established from the genetically altered non-human animal of any one of claims 27 to 29.

31. A method of screening for a compound that changes the activity of C1Gal-T2 protein, which comprises the steps of:
(a) administering a test compound to the genetically altered non-human animal of any one of claims 27 to 29, or contacting the test compound with the cell of claim 30;
(b) measuring the activity or expression level of C1Gal-T2 protein in the genetically altered non-human animal or the cell; and
(c) selecting a compound that changes the activity or expression level of C1Gal-T2 protein by comparison with activity in the absence of the test compound.
